# EUROPEAN PATENT SPECIFICATION

(11) **EP 3 809 135 B1**
(45) Date of publication and mention of the grant of the patent: **14.01.2026**
(21) Application number: 20195982.2
(22) Date of filing: 14.09.2020
(51) Int. Cl.: G01N 33/53, G01N 33/543, G01N 33/92

(54) **METHOD AND REAGENT FOR MEASURING LIPOPROTEIN'S UPTAKE ABILITY**
VERFAHREN UND REAGENZ ZUR MESSUNG DER AUFNAHMEFÄHIGKEIT VON LIPOPROTEIN
PROCÉDÉ ET RÉACTIF POUR LA MESURE DE LA CAPACITÉ D'ABSORPTION DE LIPOPROTÉINE

(30) Priority: 30.09.2019 JP 2019180871
(43) Date of publication of application: 21.04.2021
(73) Proprietor: SYSMEX CORPORATION, Kobe-shi Hyogo 651-0073 (JP)
(72) Inventor: Kubo, Takuya, Hyogo, 651-0073 (JP)
(74) Representative: Hoffmann Eitle

(56) References cited:
- EP-A1- 3 009 844
- EP-A1- 3 225 993
- EP-A1- 3 629 025
- WO-A1-2019/188297
- MURAKAMI K ET AL: "Establishment Of An Automated Assay For Cholesterol Uptake Capacity, A New Concept Of High-Density Lipoprotein Functionality", ATHEROSCLEROSIS, ELSEVIER, AMSTERDAM, NL, vol. 287, 1 August 2019 (2019-08-01), XP085789757, ISSN: 0021-9150, [retrieved on 20190805], DOI: 10.1016/J.ATHEROSCLEROSIS.2019.06.679
- HARADA A ET AL: "A potential role of cholesterol uptake capacity, a new measure for high-density lipoprotein functionality, in coronary risk stratification", ATHEROSCLEROSIS, vol. 252, 1 October 2016 (2016-10-01), XP029739147, ISSN: 0021-9150, DOI: 10.1016/J.ATHEROSCLEROSIS.2016.07.108

## Description

### CROSS REFERENCE TO RELATED APPLICATIONS

This application claims priority from prior Japanese Patent Application No. 2019-180871, filed on September 30, 2019, entitled "Method and reagent for measuring lipoprotein's uptake ability and reagent for stabilizing measurement of lipoprotein's uptake ability".

### FIELD OF THE INVENTION

The present invention relates to a method for measuring lipoprotein's uptake ability.

### BACKGROUND

In recent years, an index reflecting function of lipoprotein has attracted attention. Known methods for investigating the function of lipoprotein are, for example, methods described in US Patent Application Publication No. 2017/0315112 and US Patent Application Publication No. 2016/0109469. In the methods of these documents, lipoprotein's ability to uptake cholesterol is measured by contacting lipoprotein and tagged cholesterol in a sample to form a lipoprotein incorporating the tagged cholesterol and detecting a signal derived from the incorporated tagged cholesterol.
EP 3 629 025 A1 describes a method for obtaining information on subject's diabetes, including measuring lipoprotein's ability to uptake sterol in a biological sample of a subject, in which the measured value of ability to uptake sterol per unit volume of the biological sample is an indicator of diabetes onset risk.
WO 2019/188297 A1 describes a method for measuring the uptake performance of a lipoprotein in a highly accurate manner using *inter alia* a surfactant that does not include a cyclic structure.

### SUMMARY OF THE INVENTION

The scope of the present invention is defined solely by the appended claims, and is not affected to any degree by the statements within this summary.

The present invention provides a method for measuring lipoprotein's uptake ability, comprising: preparing lipoprotein incorporating labeled sterol by mixing lipoprotein in a sample with the labeled sterol, in a presence of at least one compound selected from the group consisting of: a compound comprising a hydrocarbon chain having at least one carbon-carbon unsaturated bond (excluding a sterol); and a saturated aliphatic compound having no phosphodiester bond; and measuring an ability to uptake the labeled sterol, based on a label of the labeled sterol incorporated into the lipoprotein, wherein the compound comprising the hydrocarbon chain is at least one selected from the group consisting of oleic acid amide, erucic acid amide, oleyl alcohol, palmitoleyl alcohol, cis-4-decen-1-ol, elaidyl alcohol, and oleylamine, wherein the saturated aliphatic compound is at least one selected from the group consisting of stearic acid amide and palmitic acid amide.

Further, the present invention provides a method for measuring a labeled sterol, comprising: forming a complex comprising a lipoprotein and the labeled sterol, in a presence of at least one compound selected from the group consisting of: a compound comprising a hydrocarbon chain having at least one carbon-carbon unsaturated bond (excluding a sterol); and a saturated aliphatic compound having no phosphodiester bond; and measuring the labeled sterol in the complex, wherein the compound comprising the hydrocarbon chain is at least one selected from the group consisting of oleic acid amide, erucic acid amide, oleyl alcohol, palmitoleyl alcohol, cis-4-decen-1-ol, elaidyl alcohol, and oleylamine, wherein the saturated aliphatic compound is at least one selected from the group consisting of stearic acid amide and palmitic acid amide.

Further, the present invention provides a method for measuring a labeled sterol, comprising: forming a complex comprising a lipoprotein and a labeled sterol in a presence of at least one compound selected from the group consisting of: a compound comprising a hydrocarbon chain having at least one carbon-carbon unsaturated bond (excluding a sterol); and a saturated aliphatic compound having no phosphodiester bond; binding a capture body with a label of the labeled sterol in the complex, the capture body comprising a fluorescent substance or an enzyme; and measuring a signal resulted from the fluorescent substance of the complex or from an enzymatic reaction by the enzyme of the complex, wherein the compound comprising the hydrocarbon chain is at least one selected from the group consisting of oleic acid amide, erucic acid amide, oleyl alcohol, palmitoleyl alcohol, cis-4-decen-1-ol, elaidyl alcohol, and oleylamine, wherein the saturated aliphatic compound is at least one selected from the group consisting of stearic acid amide and palmitic acid amide.

### BRIEF DESCRIPTION OF THE DRAWINGS

FIG. 1A is a view showing an example of an appearance of a measurement reagent or stabilizing reagent of this embodiment;
FIG. 1B is a view showing an example of an appearance of a reagent kit for measurement of this embodiment;

FIG. 2A is a view showing an example of an appearance of a reagent kit for measurement of this embodiment;
FIG. 2B is a view showing an example of an appearance of a reagent kit for measurement of this embodiment; and
FIG. 2C is a view showing an example of an appearance of a reagent kit for measurement of this embodiment.

### DETAILED DESCRIPTION OF THE PREFERRED EMBODIMENTS

### [1. Method for Measuring Lipoprotein's Uptake Ability]

In a method for measuring lipoprotein's uptake ability of this embodiment (hereinafter, also referred to as "measurement method"), first, lipoprotein in a sample is mixed with labeled sterol, in the presence of a compound containing a hydrocarbon chain having at least one carbon-carbon unsaturated bond (excluding a sterol), and/or a saturated aliphatic compound having no phosphodiester bond as defined in the appended claims. As a result, the lipoprotein comes into contact with the labeled sterol in the presence of the above compound, and the labeled sterol is incorporated into the lipoprotein.

Hereinafter, the "compound containing a hydrocarbon chain having at least one carbon-carbon unsaturated bond (excluding a sterol)" is also called "first additive". The "saturated aliphatic compound having no phosphodiester bond" is also called "second additive". **In** the measurement method of this embodiment, as shown in the examples below, by mixing the lipoprotein and the labeled sterol in the sample in the presence of the additive, variation of measured values of lipoprotein's uptake ability is reduced, as compared with a case of using no additive. That is, the measurement method of this embodiment makes it possible to improve reproducibility of measuring lipoprotein's uptake ability.

### (Compound containing hydrocarbon chain having at least one carbon-carbon unsaturated bond)

**In** the first additive, the hydrocarbon chain having at least one carbon-carbon unsaturated bond is at least one selected from the group consisting of oleic acid amide, erucic acid amide, oleyl alcohol, palmitoleyl alcohol, cis-4-decen-1-ol, elaidyl alcohol, and oleylamine as defined in the appended claims. The following aspects regarding the first additive are considered useful for understanding the invention. For example, a cyclic compound such as calixarene has a structure in which a hydrocarbon chain having at least one carbon-carbon unsaturated bond forms a ring in the molecule, and therefore, such a cyclic compound is excluded from the first additive. The hydrocarbon chain having at least one carbon-carbon unsaturated bond may have a cyclic structure in the chain as long as it is a chain structure having a terminal. Examples of the cyclic structure include non-aromatic rings having 3 to 8 carbon atoms and aromatic rings having 6 to 12 carbon atoms. Examples of the non-aromatic ring include cyclic hydrocarbons, cyclic ethers, cyclic esters (lactones), and the like. Examples of the aromatic ring include a benzene nucleus, a naphthalene nucleus, and the like. The cyclic structure may contain one or more heteroatoms selected from N, S, O and P. However, the first additive is a compound other than sterols. For example, cholesterol, the labeled sterol used in the measurement method of this embodiment and the like are excluded from the first additive.

The hydrocarbon chain having at least one carbon-carbon unsaturated bond may be linear or branched. In this aspect, a main chain of the hydrocarbon chain having at least one carbon-carbon unsaturated bond has, for example, 8 or more and 30 or less carbon atoms, preferably 10 or more and 26 or less carbon atoms, and more preferably 14 or more and 22 or less carbon atoms. The main chain of the hydrocarbon chain having at least one carbon-carbon unsaturated bond refers to a chain having a largest number of carbons, which is determined when naming the first additive according to International Union of Pure and Applied Chemistry (IUPAC) nomenclature.

In the first additive, the number of carbon-carbon unsaturated bonds contained in the hydrocarbon chain may be one or plural. The carbon-carbon unsaturated bond may be a double bond or a triple bond. When there are multiple carbon-carbon unsaturated bonds, the hydrocarbon chain of the first additive may contain both double and triple bonds.

The first additive preferably has at least one hydrophilic group. A position of the hydrophilic group is not particularly limited, but it is preferable that the hydrocarbon chain having at least one carbon-carbon unsaturated bond has the hydrophilic group. The hydrophilic group may be contained in either a main chain or a side chain of the hydrocarbon chain. Examples of the hydrophilic group include an amide group, a hydroxyl group, an amino group, a carboxyl group, a sulfo group, a thiol group, a carbonyl group, an ester group, an ether group, and a combination thereof.

The first additive may be, for example, a compound represented by a following formula (I).

R₁-CH = CH-R₂ (I)

wherein R₁ and R₂ are identical or different and are -R₃-(C=O)-NH₂, -R₃-OH, - R₃-NH₂, -R₃-(C=O)-OH, -R₃-SO₂-OH, -R₃-SH, -R₃-O-(C=O)-OH, -R₃-NH-(C=O)-NH₂, a substituted or unsubstituted alkyl group whose main chain has 1 or more and 28 or less carbon atoms, or a hydrogen atom, provided that when R₁ is an alkyl group or a hydrogen atom, R₂ is other than the alkyl group and the hydrogen atom,
the sum of carbon numbers of main chains of R₁ and R₂ is 6 or more and 28 or less, and
R₃ is an atomic bonding, or a substituted or unsubstituted alkylene group whose main chain has 1 or more and 28 or less carbon atoms.

In R₁ and R₂, the substituted or unsubstituted alkyl group whose main chain has 1 or more and 28 or less carbon atoms may have a branched chain (side chain). The main chain refers to a longest carbon chain in the above alkyl group. In the above alkyl group, the main chain having 1 or more and 28 or less carbon atoms refers to a moiety designated as a linear alkyl group selected from the group consisting of methyl, ethyl, propyl, butyl, pentyl, hexyl, heptylene, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, and octacosyl. When R₁ and/or R₂ is a substituted alkyl group whose main chain has 1 or more and 28 or less carbon atoms, a substituent is selected so that a linear carbon chain having 1 or more and 28 or less carbon atoms becomes a main chain.

In R₃, the atomic bonding refers to a direct bonding without intervening any other atom. In R₃, the substituted or unsubstituted alkylene group whose main chain has 1 or more and 28 or less carbon atoms may have a branched chain (side chain). The main chain refers to a longest carbon chain in the above alkylene group. In the above alkylene group, the main chain having 1 or more and 28 or less carbon atoms refers to a moiety designated as a linear alkylene group selected from the group consisting of methylene, ethylene, propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, icosylene, henicosylene, docosylene, tricosylene, tetracosylene, pentacosylene, hexacosylene, heptacosylene, heptacosylene, and octacosylene. When R₃ is a substituted alkylene group whose main chain has 1 or more and 28 or less carbon atoms, a substituent is selected so that a linear carbon chain having 1 or more and 28 or less carbon atoms becomes a main chain.

Examples of the substituent in R₁, R₂ and R₃ include a cyano group, an alkoxy group, =O, =S, -NO₂, -SH, halogen, a haloalkyl group, a heteroalkyl group, a carboxyalkyl group, a thioether group, and the like. Each of R₁, R₂ and R₃ may have a plurality of substituents. Halogen represents fluorine, chlorine, bromine, or iodine. Alkoxy represents an -O-alkyl group. The alkyl group in the substituent is a linear or branched saturated aliphatic hydrocarbon group having 1 or more and 5 or less carbon atoms, and preferably 1 or 2 carbon atoms.

The compound represented by the formula (I) may be in cis form or trans form. In this aspect, examples of the first additive represented by the formula (I) include linear unsaturated aliphatic amides, unsaturated aliphatic alcohols and unsaturated aliphatic amines having one carbon-carbon double bond and having 8 or more and 30 or less carbon atoms. Specific examples thereof include cis-4-decenoic acid amide, palmitoleic acid amide, oleic acid amide, elaidic acid amide, erucic acid amide, cis-4-decen-1-ol, palmitoleyl alcohol, oleyl alcohol, elaidyl alcohol, erucyl alcohol, cis-4-decenamine, palmitoleylamine, oleylamine, trans-9-octadecenamine, cis-13-docosenamine, and the like. The first additive is at least one selected from the group consisting of oleic acid amide, erucic acid amide, oleyl alcohol, palmitoleyl alcohol, cis-4-decen-1-ol, elaidyl alcohol and oleylamine as defined in the appended claims.

### (Saturated aliphatic compound having no phosphodiester bond)

The second additive is an aliphatic compound having no phosphodiester bond and having no carbon-carbon unsaturated bond selected from the group consisting of stearic acid amide and palmitic acid amide as defined in the appended claims. The aliphatic compound refers to a compound other than aromatic compounds.

The following aspects regarding the second additive are considered useful for understanding the invention. Since the second additive has no phosphodiester bond, for example, phospholipids, saturated aliphatic compounds having a structure similar to phospholipids and the like are excluded from the second additive. The second additive may be a chain compound having no cyclic structure in the molecule or a cyclic compound. Examples of the cyclic structure include a non-aromatic ring having 3 to 8 carbon atoms. Examples of the non-aromatic ring include cyclic hydrocarbons, cyclic ethers, lactones, and the like. The cyclic structure may contain one or more heteroatoms selected from N, S, O and P.

As the second additive, a saturated aliphatic compound whose main chain has 8 or more and 30 or less carbon atoms is preferred. The main chain of the saturated aliphatic compound as the second additive refers to a chain having a largest number of carbons, which is determined when naming the second additive according to the IUPAC nomenclature. Examples of the saturated aliphatic compound include saturated aliphatic amides, saturated aliphatic alcohols and saturated aliphatic amines having 8 or more and 30 or less carbon atoms in the main chain. The preferred second additive is a linear saturated aliphatic amide, saturated aliphatic alcohol or saturated aliphatic amine having 8 or more and 30 or less carbon atoms.

Examples of the linear saturated aliphatic amide having 8 or more and 30 or less carbon atoms include octanoic acid amide, nonanoic acid amide, decanoic acid amide, undecanoic acid amide, dodecanoic acid amide, tetradecanoic acid amide, hexadecanoic acid amide (palmitic acid amide), octadecanoic acid amide (stearic acid amide), icosanoic acid amide, docosanoic acid amide, tetracosanoic acid amide, hexacosanoic acid amide, octacosanoic acid amide, triacontanic acid amide, and the like. Examples of the linear saturated aliphatic alcohol having 8 or more and 30 or less carbon atoms include n-octanol, n-nonanol, n-decanol, n-undecanol, n-dodecanol, n-tetradecanol, n-hexadecanol, n-octadecanol, n-icosanol, n-docosanol, n-tetracosanol, n-hexacosanol, n-octacosanol, n-triacontanol, and the like. Examples of the linear saturated aliphatic amine having 8 or more and 30 or less carbon atoms include octylamine, nonylamine, decylamine, undecylamine, dodecylamine, tetradecylamine, hexadecylamine, octadecylamine, icosylamine, docosylamine, tetracosylamine, hexacosylamine, octacosylamine, triacontylamine, and the like. The second additive is selected from stearic acid amide or palmitic acid amide as defined in the appended claims.

### (Sample containing lipoprotein)

In this embodiment, the sample is not particularly limited as long as it contains a mammalian lipoprotein, and preferably a human lipoprotein. Examples of the sample include blood samples. Examples of the blood samples include blood (whole blood), plasma, serum, and the like. In this embodiment, the sample containing lipoprotein may be separated or fractionated by a known method such as ultracentrifugation or polyethylene glycol (PEG) precipitation to obtain a fraction containing a predetermined lipoprotein. The fraction containing a predetermined lipoprotein thus obtained may be used as a sample containing lipoprotein.

Lipoproteins may be any of high density lipoprotein (HDL), low density lipoprotein (LDL), medium density lipoprotein (IDL), very low density lipoprotein (VLDL), or chylomicron (CM). HDL is lipoprotein with a density of 1.063 g/mL or more. LDL is lipoprotein with a density of 1.019 g/mL or more and less than 1.063 g/mL. IDL is lipoprotein with a density of 1.006 g/mL or more and less than 1.019 g/mL. VLDL is lipoprotein with a density of 0.95 g/mL or more and less than 1.006 g/mL. CM is lipoprotein with a density of less than 0.95 g/mL. In this embodiment, it is preferable to measure HDL's ability to uptake sterol.

Lipoproteins are known to esterify and incorporate cholesterol. When the labeled cholesterol described later is used as the labeled sterol, a fatty acid required for esterification reaction of cholesterol with lipoprotein or a composition containing the same (for example, liposome) may be added to a sample. The fatty acid or the composition containing the same may be added to any of the sample dilution reagent described later, a solution containing labeled sterol, and a liquid reagent containing a first additive and/or a second additive.

In this embodiment, the sample containing lipoprotein may be diluted. For example, in order to adjust lipoprotein concentration, the above blood sample or a liquid obtained by diluting a fraction containing a predetermined lipoprotein can be used as a sample. An aqueous medium or a sample dilution reagent can be used for diluting the sample. The aqueous medium is not particularly limited as long as it does not inhibit an uptake reaction by lipoproteins. Examples of the aqueous medium include water, physiological saline solutions, buffer solutions, and the like. Examples of the buffer solution include phosphate buffered saline (PBS), Tris-HCl, Good buffers, and the like. The sample dilution reagent refers to a liquid containing a substance useful in measuring lipoprotein's ability to uptake sterol in the above aqueous medium. Examples of the substance useful for measurement include surfactants having no cyclic structure, cyclic oligosaccharides, components that bind to a lipoprotein different from the lipoprotein to be measured, blocking agents, and the like.

The concentration of apolipoprotein that is a component of lipoprotein is an indicator of the concentration of lipoprotein in the biological sample. In this embodiment, the lipoprotein concentration in the sample may be adjusted by diluting the sample containing lipoprotein based on the apolipoprotein concentration. The concentration of apolipoprotein can be measured by known immunoassays (for example, immunoturbidimetry). As the concentration of apolipoprotein, the concentration of ApoAI is particularly preferred. The measurement of apolipoprotein is preferably performed separately from the measurement of ability to uptake sterol of this embodiment, by taking a part of the sample containing lipoprotein to use it as a measurement sample.

In this embodiment, the sample containing lipoprotein may be diluted in the presence of the first additive and/or the second additive. For example, the sample containing lipoprotein may be diluted with a liquid containing the first additive and/or the second additive in the above aqueous medium or sample dilution reagent. By mixing the sample diluted as described above with labeled sterol described later, the lipoprotein and the labeled sterol come into contact with each other in the presence of the first additive and/or the second additive.

In this embodiment, the sample containing lipoprotein may be diluted in the presence of a surfactant having no cyclic structure. For example, the sample containing lipoprotein may be diluted with a sample dilution reagent containing a surfactant having no cyclic structure. A sterol uptake reaction by lipoproteins is promoted by using a surfactant having no cyclic structure. In addition, the surfactant having no cyclic structure also plays the role of blocking agent. Therefore, an animal-derived protein generally used as a blocking agent in immunological measurements, such as bovine serum albumin (BSA), may not be used. The surfactant having no cyclic structure refers to a surfactant which is an acyclic compound. Details of the surfactant having no cyclic structure will be described later.

In this embodiment, the sample containing lipoprotein may be diluted in the presence of a cyclic oligosaccharide. For example, the sample containing lipoprotein may be diluted with a sample dilution reagent containing a cyclic oligosaccharide. Examples of the cyclic oligosaccharide include cyclodextrin, hydroxypropyl cyclodextrin, and the like. The cyclic oligosaccharide functions as a blocking agent by including cholesterol derived from the sample.

In this embodiment, the sample containing lipoprotein may be diluted in the presence of a component that binds to a lipoprotein different from the lipoprotein to be measured. For example, the sample containing lipoprotein may be diluted with a sample dilution reagent containing the component. Examples of the component that binds to a lipoprotein different from the lipoprotein to be measured include calixarene and the like. Calixarene binds to LDL, VLDL and CM but not to HDL. When measuring uptake ability by HDL, addition of calixarene to a sample can mask LDL, VLDL and CM derived from the sample.

In this embodiment, the sample containing lipoprotein may be diluted in the presence of a blocking agent to prevent nonspecific adsorption of the labeled sterol. For example, the sample containing lipoprotein may be diluted with a sample dilution reagent containing such a blocking agent. The addition of the blocking agent suppresses, for example, nonspecific adsorption of the labeled sterol to the solid phase or capture body described later. Examples of the blocking agent for preventing nonspecific adsorption of the labeled sterol include 2-methacryloyloxyethyl phosphoryl choline type polymer. This polymer may be a homopolymer of 2-methacryloyloxyethyl phosphoryl choline or a copolymer with another monomer. As another monomer, an acrylic ester or methacrylic ester having an alkyl group having 1 to 20 carbon atoms as a hydrophobic group is preferable. The 2-methacryloyloxyethyl phosphoryl choline type polymer is commercially available. Examples thereof include a series of Lipidure (trademark) of NOF Corporation, and among them, Lipidure-BL203 and Lipidure-SF08 are particularly preferable.

### (Labeled sterol)

The labeled sterol is sterol having a labeling substance. Hereinafter, the labeling substance possessed by the labeled sterol is also referred to as a "first label". Sterol used for the labeled sterol is not particularly limited as long as they are incorporated into lipoprotein. Examples of the sterol include cholesterol and derivatives thereof. Examples of the cholesterol derivatives include precursors of bile acids, precursors of steroids, and the like. Specifically, 3β-hydroxy-Δ5-cholenoic acid, 24-amino-5-colen-3β-ol and the like are preferable.

In this embodiment, the labeled sterol is preferably cholesterol having a labeling substance (hereinafter, also referred to as "labeled cholesterol"). A cholesterol moiety in the labeled cholesterol may have a structure of naturally occurring cholesterol, or a structure of cholesterol obtained by removing one or more methylene groups and/or methyl groups from an alkyl chain bonded to C17 position of naturally occurring cholesterol (also called norcholesterol).

It is preferable to use labeled cholesterol which is esterified by lipoproteins, since lipoproteins incorporate cholesterol by esterification. In this embodiment, the labeled cholesterol is esterified by lecithin-cholesterol acyltransferase (LCAT) of biological origin contained in the sample when it is mixed with the biological sample. Methods for confirming esterification of labeled cholesterol by lipoproteins themselves are known in the art and can be routinely performed by those skilled in the art.

The first label is not particularly limited as long as it is a labeling substance that makes it possible to detect the labeled sterol incorporated into lipoprotein. The first label may be, for example, a tag that is itself to be detected, or may be a substance that generates a detectable signal (hereinafter, also referred to as a "signal generating substance").

The tag as the first label is not particularly limited as long as it does not inhibit uptake of sterol by lipoproteins, and a substance capable of specifically binding to the tag is present or obtained. Hereinafter, sterol added with a tag as the first label is also referred to as "tagged sterol". Similarly, cholesterol added with a tag as the first label is also referred to as "tagged cholesterol". Tagged cholesterol itself is known in the art and is described, for example, in Patent Document 1 (US 2017/0315112 A1).

Examples of the tagged cholesterol include tagged cholesterol represented by a following formula (II). 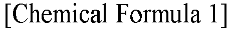 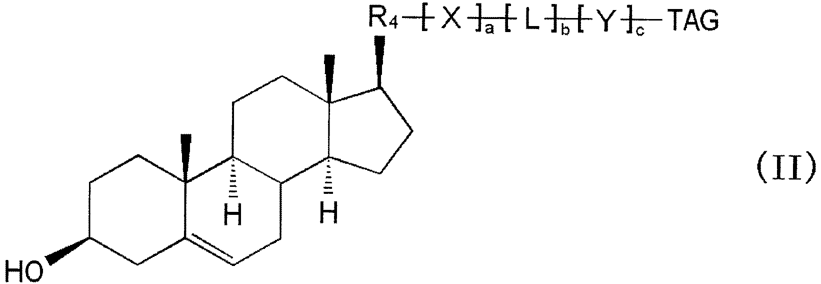
wherein R₄ is an alkylene group having 1 to 6 carbon atoms which optionally have a methyl group,
X and Y are identical or different, and are represented by -R₅-NH-, -NH-R₅-, - R₅-(C=O)-NH-, -(C=O)-NH-R₅-, -R₅-NH-(C=O)-, -NH-(C=O)-R₅-, -R₅-(C=O)-, - (C=O)-R₅-, -R₅-(C=O)-O-, -(C=O)-O-R₅-, -R₅-O-(C=O)-, -O-(C=O)-R₅-, -R₅-(C=S)-NH-, -(C=S)-NH-R₅-, -R₅-NH-(C=S)-, -NH-(C=S)-R₅-, -R₅-O-, -O-R₅-, -R₅-S-, or -S-R₅-, and each R₅ is independently an atomic bonding, an alkylene group having 1 to 10 carbon atoms which optionally have a substituent, or an arylene group or heteroarylene group having 6 to 12 carbon atoms which optionally have a substituent, or a cycloalkylene group or heterocycloalkylene group having 3 to 8 carbon atoms which optionally have a substituent,
L is represented by -(CH₂)_{d}-[R₆-(CH₂)ₑ]_{f}- or -[(CH₂)ₑ-R₆]_{f}-(CH₂)_{d}-, and R₆ is an oxygen atom, a sulfur atom, -NH-, -NH-(C=O)- or -(C=O)-NH-,
TAG is a tag,
a and c are identical or different and are an integer of 0 to 6,
b is 0 or 1,
d and e are identical or different and are an integer of 0 to 12, and
f is an integer of 0 to 24.

In the formula (II), when a, b and c are all 0, the tagged cholesterol represented by the formula has no linker, and the tag and the cholesterol moiety are bonded directly. In the formula (II), when any one of a, b and c is not 0, the tagged cholesterol represented by the formula has a linker (-[X]ₐ-[L]_{b}-[Y]_{c}-) between the tag and the cholesterol moiety. It is believed that the linker further facilitates the bonding between the tag exposed on the outer surface of the lipoprotein and the capture body. Each substituent in the formula (II) will be described below.

R₄ may have an alkylene group having 1 or more and 6 or less carbon atoms as a main chain, and optionally have a methyl group at any position. R₄ corresponds to an alkyl chain bonded to C17 position of naturally occurring cholesterol. In this embodiment, when R₄ has 1 or more and 5 or less carbon atoms, R₄ preferably has a methyl group at the position of C20 in the naturally occurring cholesterol. When R₄ has 6 carbon atoms, R₄ preferably has the same structures as alkyl chains at C20 to C27 positions of the naturally occurring cholesterol.

[X]ₐ corresponds to a connecting moiety between R₄ and L, [Y]_{c} or the tag. [Y]_{c} corresponds to a connecting moiety between R₄, [X]ₐ or L and the tag. X and Y are determined according to the type of reaction that bonds between the cholesterol moiety and the linker and the type of reaction that bonds between the linker and the tag.

In R₅, the atomic bonding refers to a direct bonding without intervening any other atom. When R₅ is an alkylene group having 1 to 10 carbon atoms, examples of the alkylene group include methylene, ethylene, propylene, isopropylene, butylene, isobutylene, pentylene, neopentylene, hexylene, heptylene, octylene, 2-ethylhexylene, nonylene, and decylene groups. Among them, an alkylene group having 1 or more and 4 or less carbon atoms is preferred. When R₅ is an alkylene group having a substituent, the above number of carbon atoms does not include the number of carbon atoms in the substituent.

When R₅ is an arylene group or a heteroarylene group, the group should be an aromatic ring having 6 or more and 12 or less carbon atoms which may contain one or more hetero atoms selected from N, S, O, and P. Examples of the group include phenylene, naphthylene, biphenylylene, furanylene, pyrrolene, thiophenylene, triazolene, oxadiazolene, pyridylene, and pyrimidylene groups. When R₅ is an arylene group or a heteroarylene group having a substituent, the above number of carbon atoms does not include the number of carbon atoms in the substituent.

When R₅ is a cycloalkylene group or a heterocycloalkylene group, the group should be a non-aromatic ring having 3 or more and 8 or less carbon atoms which may include one or more hetero atoms selected from N, S, O, and P. Examples of the group include cyclopropylene, cyclobutylene, cyclopentylene, cyclohexylene, cycloheptylene, cyclooctylene, pyrrolidinylene, piperidinylene, and morpholinylene groups. When R₅ is a cycloalkylene group or a heterocycloalkylene group having a substituent, the above number of carbon atoms does not include the number of carbon atoms in the substituent.

Examples of the substituent in R₅ include hydroxyl, cyano, alkoxy, =O, =S, - NO₂, -SH, halogen, haloalkyl, heteroalkyl, carboxyalkyl, amine, amide, and thioether groups. R₅ may have a plurality of the substituents. Halogen represents fluorine, chlorine, bromine, or iodine. Alkoxy represents an -O-alkyl group, and the alkyl group is a linear or branched saturated aliphatic hydrocarbon group having 1 or more and 5 or less carbon atoms, and preferably 1 or 2 carbon atoms.

Preferably, a and c are both 1, and X and Y are identical or different and are - (C=O)-NH- or -NH-(C=O)-.

L corresponds to a spacer and has a polymer structure that adds a predetermined length to the linker. It is preferable that the polymer structural moiety does not inhibit the uptake of cholesterol by lipoproteins, and that the linker moiety is hardly incorporated into lipoproteins. The polymers include hydrophilic polymers such as polyethylene glycol (PEG). In a preferred embodiment, L is a structure represented by -(CH₂)_{d}-[O-(CH₂)ₑ]_{f}- or -[(CH₂)ₑ-O]_{f}-(CH₂)_{d}-. d and e are identical or different and are an integer of 0 to 12, preferably an integer of 2 to 6, and more preferably both 2. f is an integer of 0 to 24, preferably an integer of 2 to 11, and more preferably an integer of 4 to 11.

The tag may be any of naturally occurring substances and synthesized substances, and examples thereof include compounds, peptides, proteins, nucleic acids, a combination thereof, and the like. The compound may be a labeling compound known in the art as long as a substance capable of specifically binding to the compound is present or obtained, and examples thereof include dye compounds and the like.

It is known in the art that cholesterol is esterified to increase its lipid solubility and promote its uptake by lipoproteins. The tag attached to cholesterol may be a lipid soluble or hydrophobic substance.

Examples of combinations of a tag and a substance capable of specifically binding to the tag include an antigen and an antibody that recognizes the antigen, a hapten and an anti-hapten antibody, a peptide or a protein and an aptamer that recognizes them, a ligand and a receptor thereof, oligonucleotides and oligonucleotides having complementary strands thereof, biotins (including biotin, and biotin analogs such as desthiobiotin) and avidins (including avidin, and avidin analogs such as streptavidin and tamavidin (registered trademark)), histidine tag (a peptide containing histidine of 6 to 10 residues) and nickel, glutathione-S-transferase (GST) and glutathione, and the like. The antigen as a tag may be a peptide tag and a protein tag known in the art, and examples thereof include FLAG (registered trademark), hemagglutinin (HA), Myc protein, green fluorescent protein (GFP), and the like. Examples of the hapten as a tag include 2,4-dinitrophenol and the like.

Examples of the tagged sterol include sterol added with a structure represented by a following formula (III): 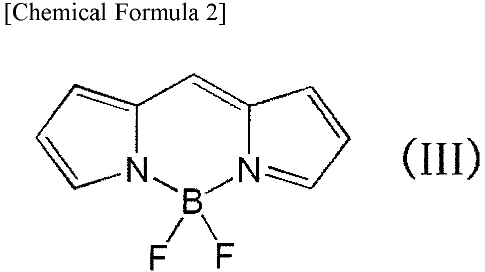 or a structure represented by a following formula (IV): 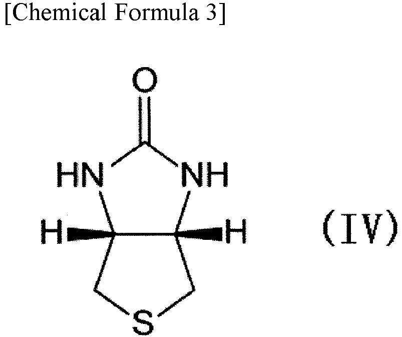 as a tag. The structure represented by the formula (III) is a boron dipyrromethene (BODIPY (registered trademark)) backbone. The structure represented by the formula (IV) shows a part of biotin. Sterol added with a structure represented by the formula (III) or (IV) as a tag is preferable since capture bodies for these tags are generally available. Sterol added with a 2,4-dinitrophenyl (DNP) group as a tag is also preferable because anti-DNP antibody is commercially available.

Examples of the tagged cholesterol having no linker include fluorescently labeled cholesterol (23-(dipyrrometheneboron difluoride)-24-norcholesterol, CAS No: 878557-19-8) represented by a following formula (V).

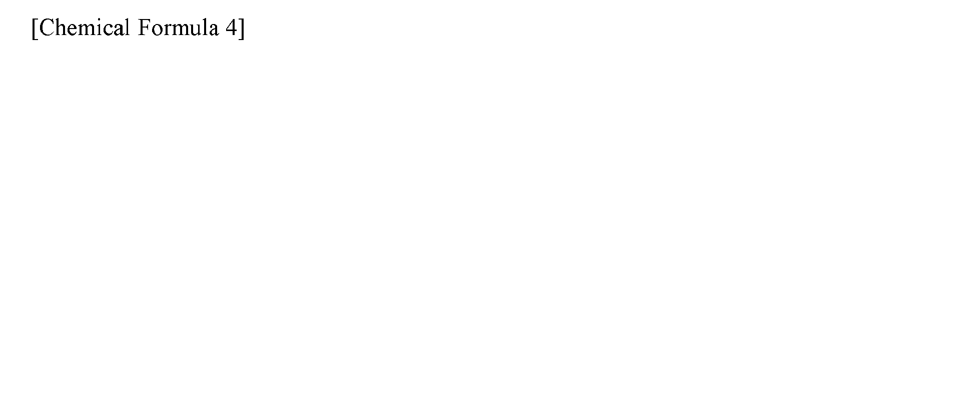

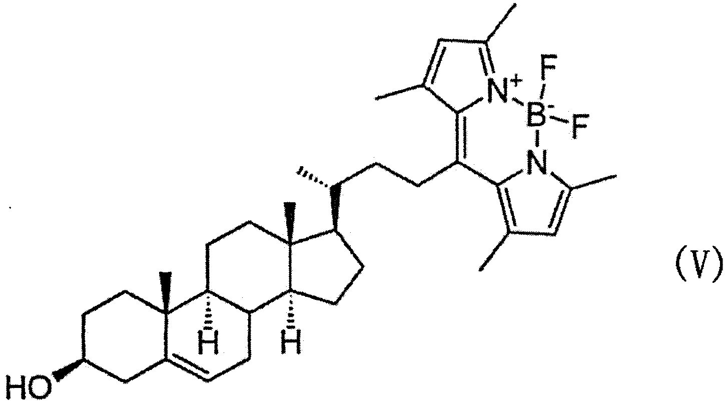

This fluorescently labeled cholesterol is sold by Avanti Polar Lipids, Inc. under the trade name TopFluor Cholesterol. In the fluorescently labeled cholesterol represented by the formula (V), the tag (the fluorescent moiety having BODIPY backbone structure) is directly bonded at C23 position of cholesterol. As a capture body that specifically binds to the fluorescent moiety having BODIPY backbone structure, an anti-BODIPY antibody (BODIPY FL Rabbit IgG Fraction, A-5770, Life Technologies Corporation) is commercially available.

The tagged cholesterol in which the tag is bound via a linker includes a biotin-tagged cholesterol represented by a following formula (VI). 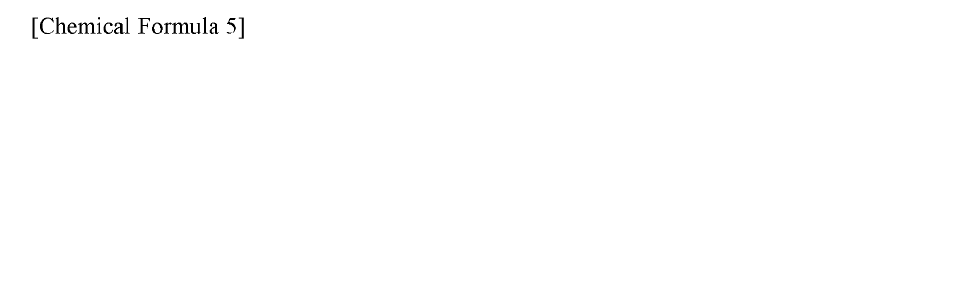 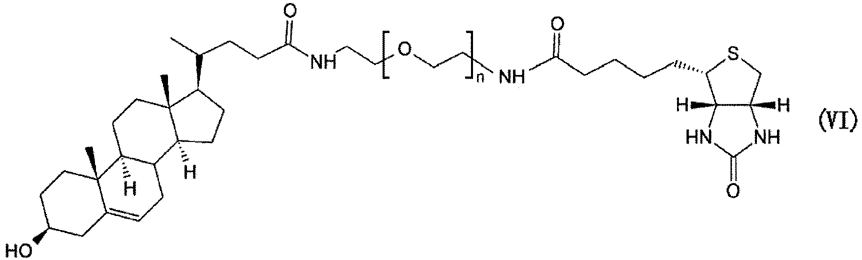 wherein, n is an integer of 0 to 24, preferably an integer of 2 to 11, and more preferably an integer of 4 to 11.

In the tagged cholesterol, the tag (the biotin moiety represented by the formula (IV)) is bonded to the cholesterol moiety via the linker (polyethylene glycol chain). As a capture body that specifically binds to the biotin moiety, avidin or streptavidin is suitable. Avidin or streptavidin to which a labeling substance such as horseradish peroxidase (HRP) or alkaline phosphatase (ALP) is bound is also commercially available.

The tagged cholesterol in which the tag is bound via a linker includes DNP-added cholesterol represented by a following formula (VII).

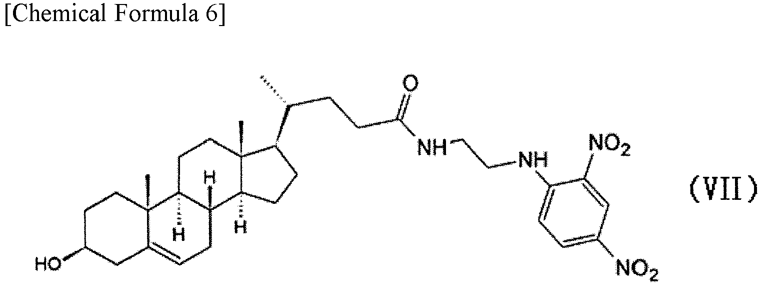

In the tagged cholesterol, DNP is bound to the cholesterol moiety via a linker (-(C=O)-NH-CH₂-CH₂-NH-). As a capture body that specifically binds to DNP, an anti-DNP antibody is suitable. Anti-DNP antibodies to which a labeling substance such as HRP or ALP is bound is also commercially available.

Although the bonding form of the sterol moiety and the tag is not particularly limited, the sterol moiety and the tag may be bonded, or the sterol moiety and the tag may be bonded via a linker. Although the bonding means is not particularly limited, for example, a crosslink by utilizing a functional group is preferred because it is convenient. Although the functional group is not particularly limited, an amino group, a carboxyl group and a sulfhydryl group are preferred because commercially available crosslinkers can be used.

Since cholesterol has no functional group in the alkyl chain bonded to the C17 position, a cholesterol derivative having a functional group in the alkyl chain is preferably used in the addition of the tag. Examples of the cholesterol derivative include precursors of bile acid, precursor of steroids, and the like. Specifically, 3β-hydroxy-Δ5-cholenoic acid, 24-amino-5-colen-3β-ol and the like are preferable. The functional group of the tag varies depending on the type of the tag. For example, when a peptide or a protein is used as the tag, an amino group, a carboxyl group and a sulfhydryl group (SH group) can be used. When biotin is used as the tag, a carboxyl group in the side chain can be used. The linker is preferably a polymer compound having functional groups at both terminals. When biotin is added as the tag, a commercially available biotin labeling reagent may be used. The reagent contains biotin to which various length of spacer arms (for example, PEG) having a functional group such as an amino group are bound at the terminal.

Examples of the signal generating substance as the first label include fluorescent substances, luminescent substances, color-developing substances, radioactive isotopes, and the like. Among them, fluorescent substances are particularly preferred. Preferably, the fluorescent substance contains a fluorophore having a polar structure. In the art, the fluorescent substance containing a fluorophore having a polar structure itself is known.

Sterol labeled with a signal generating substance can be produced by adding the substance to sterol by a known method. In sterol, a position to which a signal generating substance is added is not particularly limited, and can be appropriately determined according to the type of signal generating substance to be used. Although the bonding form of the sterol and the signal generating substance is not particularly limited, it is preferable that both are directly bonded via a covalent bond.

Examples of the sterol containing a fluorescent substance include a fluorescently labeled sterol containing a fluorophore having a **BODIPY** backbone structure represented by the formula (III). In this embodiment, commercially available fluorescently labeled sterol may be used. Examples of the sterol containing a fluorophore having a **BODIPY** backbone structure include fluorescently labeled cholesterol represented by the formula (V). The fluorescently labeled cholesterol represented by the formula (V) generates a fluorescence signal at a wavelength of 525 to 550 nm by an excitation wavelength of 470 to 490 nm.

### (Preparing lipoprotein incorporating labeled sterol)

In this preparing, mixing of the lipoprotein and the labeled sterol in the presence of a first additive and/or a second additive can be performed, for example, by mixing a sample containing lipoprotein, a solution containing labeled sterol, and a liquid reagent containing a first additive and/or a second additive. The order of mixing is not particularly limited. In this case, examples of the liquid reagent include a liquid containing the first additive and/or the second additive in the above aqueous medium or sample dilution reagent. Alternatively, as the liquid reagent, a liquid containing the first additive and/or the second additive in the solution containing labeled sterol may be used. In this case, the mixing is performed by mixing a sample containing lipoprotein and a liquid reagent containing a first additive and/or a second additive and labeled sterol. In this preparing, the lipoprotein starts to incorporate the labeled sterol in the presence of the first additive and/or the second additive, and lipoprotein incorporating labeled sterol is obtained.

The conditions of temperature and time in mixing are not particularly limited. For example, a mixed solution of a sample, labeled sterol and a liquid reagent may be incubated at 20 to 48°C, preferably 25 to 42°C, for 10 seconds to 24 hours, preferably 1 minutes to 2 hours. During the incubation, the mixed solution may be allowed to stand, or may be stirred or shaken.

The addition amount of the first additive and/or the second additive can be appropriately determined. For example, the concentration of the first additive and/or the second additive in the mixed solution obtained in the preparing the lipoprotein incorporating labeled sterol is 1 µM or more and 1000 µM or less, and preferably 13 µM or more and 352 µM or less. The mixed solution obtained in the preparing is a liquid containing lipoprotein, labeled sterol, and the first additive and/or the second additive. The mixed solution is, for example, a mixed solution of a sample containing lipoprotein, a solution containing labeled sterol, and a liquid reagent containing a first additive and/or a second additive. Alternatively, the mixed solution obtained in the preparing may be a mixed solution of a sample containing lipoprotein and a liquid reagent containing a first additive and/or a second additive and labeled sterol. When the concentration of the first additive and/or the second additive in the mixed solution obtained in the preparing is within the above range, variation of measured values is further reduced.

The addition amount of the labeled sterol is not particularly limited, but may be added in a slight excess so that the labeled sterol is not depleted. For example, the concentration of the labeled sterol in the mixed solution obtained in the preparation of the lipoprotein incorporating labeled sterol is 0.1 µM or more and 30 µM or less, and preferably 1 µM or more and 10 µM or less.

In this embodiment, the mixing of the lipoprotein in the sample and the labeled sterol may be performed in the presence of a surfactant having no cyclic structure. For example, a surfactant having no cyclic structure may be added to the solution containing labeled sterol or the above liquid reagent. The addition amount of the surfactant having no cyclic structure can be appropriately set. For example, the concentration of the surfactant in the mixed solution obtained in the preparing the lipoprotein incorporating labeled sterol is 0.001% (v/v) or more and 0.5% (v/v) or less, and preferably 0.01% (v/v) or more and 0.1% (v/v) or less.

**In** this embodiment, the mixing of the lipoprotein in the sample and the labeled sterol may be performed in the presence of the cyclic oligosaccharide and surfactant having no cyclic structure. The contact between the lipoprotein in the sample and the labeled sterol may be performed in the presence of a component that binds to a lipoprotein different from the lipoprotein to be measured and a surfactant having no cyclic structure. For example, a component that binds to a cyclic oligosaccharide and/or a lipoprotein different from the lipoprotein to be measured may be added to the solution containing labeled sterol or the above liquid reagent.

**In** this embodiment, the mixing of the lipoprotein in the sample and the labeled sterol may be performed in the presence of a blocking agent for preventing nonspecific adsorption of the labeled sterol. For example, the blocking agent may be added to the solution containing labeled sterol or the above liquid reagent. As the blocking agent, 2-methacryloyloxyethyl phosphoryl choline type polymer is preferred.

### (Surfactant having no cyclic structure)

The surfactant having no cyclic structure can be appropriately selected from nonionic surfactants, cationic surfactants, anionic surfactants and amphoteric surfactants, but is preferably a nonionic surfactant. Nonionic surfactants having no cyclic structure are not particularly limited, and examples thereof include polyalkylene glycol ethylene oxide adduct, polyoxyethylene alkyl ether, polyoxyethylene polyoxypropylene alkyl ether, polyoxyalkylene dialkyl ether, polyoxyalkylene alkenyl ether, polyoxyethylene branched alkyl ether, polyoxyethylene polyoxypropylene alkyl ether, polyoxyalkylene ether, polyethylene glycol fatty acid ester, glycerin fatty acid ester, polyglycerol fatty acid ester, propylene glycol fatty acid ester, polypropylene glycol fatty acid ester, polyoxyethylene coconut fatty acid glyceryl, polyoxyethylene hardened castor oil, polyoxyethylene castor oil, polyoxyethylene triisostearic acid, polyoxyethylene sorbitol fatty acid ester, polyoxyethylene alkylamine, polyoxyethylene polyoxypropylene alkylamine, polyoxyethylene alkyl propylene diamine, fatty acid diethanolamide, polyoxyethylene fatty acid monoethanolamide, and the like. The nonionic surfactant having no cyclic structure may be used alone or in combination of two or more.

Among the nonionic surfactants, polyoxyethylene alkyl ether and polyalkylene glycol ethylene oxide adduct are preferable. Examples of the polyoxyethylene alkyl ether include polyoxyethylene lauryl ether, polyoxyethylene cetyl ether, polyoxyethylene stearyl ether, polyoxyethylene behenyl ether, and the like. In this embodiment, polyoxyethylene lauryl ether is particularly preferred. Polyoxyethylene lauryl ether is commercially available, and examples thereof include NONION K-230 and NONION K-220 of NOF Corporation, EMULGEN 123P and EMULGEN 130K of Kao Corporation, and the like.

In this embodiment, as the polyalkylene glycol ethylene oxide adduct, a block copolymer is preferable, and a triblock copolymer of ethylene oxide and propylene oxide is more preferable. As a nonionic surfactant of the block copolymer, a compound represented by a following formula (VIII) is particularly preferable. 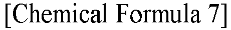 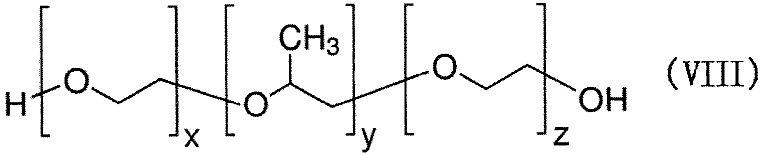 wherein x, y and z are identical or different and an integer of 1 or more, and the molecular weight of the polypropylene oxide moiety is 2750 or less.

The compounds of the formula (VIII) are also called polypropylene glycol ethylene oxide adducts, polyoxyethylene polyoxypropylene glycols, polyoxyethylene polyoxypropylene block polymers, or pluronic (trademark)-type nonionic surfactants.

In the formula (VIII), the molecular weight of the polypropylene oxide moiety (hereinafter also referred to as "PPO") refers to a molecular weight of a moiety represented by -(O-CH(CH₃)-CH₂)_{y}-, and a molecular weight calculated from the molecular structural formula. In this embodiment, the molecular weight of PPO is preferably 900 or more and 2750 or less, more preferably 950 or more and 2750 or less, and particularly preferably 950 or more and 2250 or less. Because PPO in pluronic-type nonionic surfactants is relatively highly hydrophobic, the larger the molecular weight of PPO, the greater the hydrophobicity of the surfactant. Therefore, a pluronic-type nonionic surfactant having a molecular weight of PPO more than 2750 may act on lipoprotein to inhibit uptake of cholesterol.

The average molecular weight of the compound represented by the formula (VIII) is preferably 1000 to 13000. In the formula (VIII), the sum of x and z is preferably 2 to 240. In the formula (VIII), y is preferably 15 to 47, more preferably 16 to 47, and particularly preferably 16 to 39. As used herein, the "average molecular weight" is a weight average molecular weight measured by gel permeation chromatography (GPC).

Pluronic-type nonionic surfactants are commercially available, and examples thereof include the Pluronic series of BASF, the Pronon series of NOF Corporation, the Adeka Pluronic series of Asahi Denka Co., Ltd., and the like. The Pluronic series is classified by a Pluronic grid in which the molecular weight of PPO is taken on the vertical axis and the ethylene oxide (EO) content (%) is taken on the horizontal axis (for example, see Pitto-Barry A. and Barry N.P.E., Poly. Chem., 2014, vol. 5, p. 3291-3297). The Pluronic grid is provided not only from academic literature but also from various manufacturers. In this embodiment, the compound represented by the formula (VIII) may be selected based on the pluronic grid.

Examples of the pluronic-type nonionic surfactants represented by the formula (VIII) include Pluronic L31 (molecular weight of PPO: 950, EO content: 10%), Pluronic L35 (molecular weight of PPO: 950, EO content: 50%), Pluronic F38 (molecular weight of PPO: 950, EO content: 80%), Pluronic L42 (molecular weight of PPO: 1200, EO content: 20%), Pluronic L43 (molecular weight of PPO: 1200, EO content: 30%), Pluronic L44 (molecular weight of PPO: 1200, EO content: 40%), Pluronic L61 (molecular weight of PPO: 1750, EO content: 10%), Pluronic L62 (molecular weight of PPO: 1750, EO content: 20%), Pluronic L63 (molecular weight of PPO: 1750, EO content: 30%), Pluronic L64 (molecular weight of PPO: 1750, EO content: 40%), Pluronic P65 (molecular weight of PPO: 1750, EO content: 50%), Pluronic F68 (molecular weight of PPO: 1750, EO content: 80%), Pluronic L72 (molecular weight of PPO: 2050, EO content: 20%), Pluronic P75 (molecular weight of PPO: 2050, EO content: 50%), Pluronic F77 (molecular weight of PPO: 2050, EO content: 70%), Pluronic L81 (molecular weight of PPO: 2250, EO content: 10%), Pluronic P84 (molecular weight of PPO: 2250, EO content: 40%), Pluronic P85 (molecular weight of PPO: 2250, EO content: 50%), Pluronic F87 (molecular weight of PPO: 2250, EO content: 70%), Pluronic F88 (molecular weight of PPO: 2250, EO content: 80%), Pluronic L92 (molecular weight of PPO: 2750, EO content: 20%), Pluronic P94 (molecular weight of PPO: 2750, EO content: 40%), Pluronic F98 (molecular weight of PPO: 2750, EO content: 80%), and the like.

Alternatively, the triblock copolymer of ethylene oxide and propylene oxide may be a compound represented by a following formula (IX). This compound is also called a reverse pluronic-type nonionic surfactant. 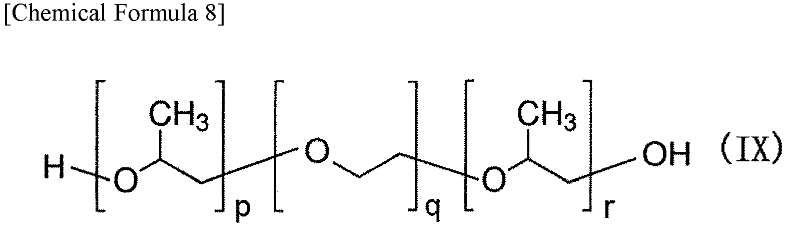 wherein p, q and r are identical or different and an integer of 1 or more, and the molecular weight of the polypropylene oxide moiety is 2750 or less.

In the formula (IX), the molecular weight of the polypropylene oxide moiety refers to a molecular weight of a moiety represented by -(O-CH(CH₃)-CH₂)ₚ- and -(O-CH(CH₃)-CH₂)ᵣ-, and a molecular weight calculated from the molecular structural formula. In this embodiment, the molecular weight of PPO is preferably 900 or more and 2750 or less, and more preferably 950 or more and 2250 or less. Pluronic-type nonionic surfactants represented by the formula (IX) are commercially available, and examples thereof include Pluronic 10R5 (molecular weight of PPO: 950, EO content: 50%) of BASF and the like.

The average molecular weight of the compound represented by the formula (IX) is preferably 1000 to 13000. In the formula (IX), q is preferably 2 to 240. In the formula (IX), the sum of p and r is preferably 15 to 47, more preferably 16 to 47, and particularly preferably 16 to 39.

### (Forming a complex of lipoprotein and first capture body)

The measurement method of this embodiment preferably includes, between the preparing the lipoprotein incorporating labeled sterol and the measuring uptake ability described later, mixing the lipoprotein incorporating labeled sterol with a first capture body that binds to the lipoprotein and forming a complex containing the lipoprotein incorporating labeled sterol and the first capture body. The contact between the lipoprotein and the first capture body causes the first capture body to bind to the lipoprotein to form a complex of the lipoprotein and the first capture body.

The first capture body is not particularly limited as long as it is a substance capable of specifically binding to a part of the surface of lipoprotein. As the first capture body, an antibody that specifically binds to lipoprotein is preferable, and an antibody that can specifically bind to apolipoprotein that is a component of lipoprotein is more preferable. Examples of the antibody include anti-ApoAI antibodies, anti-ApoAII antibodies, and the like. Among them, anti-ApoAI antibodies are particularly preferred. Commercially available anti-lipoprotein antibodies and anti-ApoAI antibodies may be used.

The anti-lipoprotein antibodies and the anti-ApoAI antibodies may be monoclonal antibodies or polyclonal antibodies. The origin of the antibody is not particularly limited, and may be an antibody derived from any mammal such as a mouse, a rat, a hamster, a rabbit, a goat, a horse, or a camel. The isotype of antibody may be any of IgG, IgM, IgE, IgA and the like and is preferably IgG. A fragment of an antibody and a derivative thereof may be used as the first capture body, and examples thereof include Fab fragments, F(ab')2 fragments, and the like.

The mixing of the lipoprotein incorporating labeled sterol and the first capture body may be performed, for example, by mixing a sample containing lipoprotein, a solution containing labeled sterol, and a solution containing the first capture body, in the presence of the first additive and/or the second additive. The order of mixing is not particularly limited. In a preferred embodiment, the lipoprotein and the labeled sterol are mixed in the presence of the first additive and/or the second additive, and then the mixing of the lipoprotein and the first capture body is performed. For example, the sample containing lipoprotein and the solution containing labeled sterol are mixed in the presence of the first additive and/or the second additive, and then the obtained mixed solution and the solution containing the first capture body are mixed. Mixing in the presence of the first additive and/or the second additive is preferably performed using the above liquid reagent. As a result, a complex of the lipoprotein incorporating labeled sterol and the first capture body is formed. The addition amount of the first capture body is not particularly limited, and can be appropriately set by those skilled in the art according to the type of the first capture body and the like.

When an anti-ApoAI antibody is used as the first capture body, the lipoprotein may be treated with an oxidizing agent before the anti-ApoAI antibody and the lipoprotein incorporating labeled sterol come in contact with each other. By the action of the oxidizing agent, reactivity of the anti-ApoAI antibody and the lipoprotein can be improved. Examples of the oxidizing agent include hydrogen peroxide, peroxynitrite, chlorine dioxide, hypochlorous acid, and the like.

There are no particular limitations on the conditions of temperature and time in the mixing of the lipoprotein and the first capture body. For example, the mixed solution may be incubated at 20 to 48°C, preferably 25 to 42°C, for 10 seconds to 24 hours, and preferably 1 minute to 2 hours. During the incubation, the mixed solution may be allowed to stand, or may be stirred or shaken.

### (Immobilization of complex on solid phase)

In this embodiment, the complex of the lipoprotein incorporating labeled sterol and the first capture body may be immobilized on a solid phase. The solid phase is preferably a solid phase capable of capturing the first capture body in the complex. Immobilization of the complex on the solid phase may be performed, for example, by mixing a sample containing lipoprotein, a solution containing labeled sterol, a solution containing the first capture body and a solid, in the presence of the first additive and/or the second additive. Alternatively, the sample containing lipoprotein, the solution containing labeled sterol, and the solution containing the first capture body are mixed, in the presence of the first additive and/or the second additive, and then the obtained mixed solution and a solid phase may be mixed. Preferably, first, the sample containing lipoprotein and the solution containing labeled sterol are mixed, subsequently the obtained mixed solution and the solution containing the first capture body are mixed, and then the obtained mixed solution and the solid phase are mixed, in the presence of the first additive and/or the second additive. Mixing in the presence of the first additive and/or the second additive is preferably performed using the above liquid reagent.

In this embodiment, the first capture body may be previously immobilized on a solid phase. For example, a sample containing lipoprotein, a solution containing labeled sterol, and a solid phase on which the first capture body is immobilized may be mixed, in the presence of the first additive and/or the second additive. Preferably, the sample containing lipoprotein and the solution containing labeled sterol are mixed, and then the obtained mixed solution and the solid phase on which the first capture body is immobilized are mixed, in the presence of the first additive and/or the second additive. Mixing in the presence of the first additive and/or the second additive is preferably performed using the above liquid reagent.

The first capture body in the complex is captured by the solid phase and immobilized on the surface of the solid phase, whereby the complex is immobilized on the solid phase. The conditions of temperature and time of mixing using a solid phase are not particularly limited. For example, the conditions may be similar to those for the mixing of the lipoprotein and the first capture body.

The type of the solid phase is not particularly limited, and examples of the solid phase include a solid phase of a material that physically adsorbs the antibody, a solid phase on which a molecule that specifically binds to the antibody is immobilized, and the like. Examples of the molecule that specifically binds to the antibody include protein A or G, an antibody (i.e., a secondary antibody) that specifically recognizes an antibody, and the like. A combination of substances interposed between the antibody and the solid phase can be used to bind them. Examples of the combination of substances include combinations of biotins and avidins, haptens and anti-hapten antibodies, and the like. For example, when the first capture body is previously modified with biotin, the first capture body can be captured by a solid phase on which avidin is immobilized.

The solid phase material can be selected from organic polymer compounds, inorganic compounds, biopolymers, and the like. Examples of the organic polymer compounds include latex, polystyrene, polypropylene, styrene-methacrylic acid copolymer, styrene-glycidyl (meth)acrylate copolymer, styrene-styrene sulfonate copolymer, methacrylic acid polymer, acrylic acid polymer, acrylonitrile butadiene styrene copolymer, vinyl chloride-acrylate copolymer, polyvinyl acetate acrylate, and the like. Examples of the inorganic compounds include magnetic bodies (iron oxide, chromium oxide, cobalt, ferrite, etc.), silica, alumina, glass, and the like. Examples of the biopolymer include insoluble agarose, insoluble dextran, gelatin, cellulose, and the like. Two or more of these may be used in combination.

The shape of the solid phase is not particularly limited, and examples thereof include a particle, a microplate, a microtube, a test tube, and the like. Among them, a particle and a microplate are preferred, and a magnetic particle and a 96-well microplates are particularly preferred. When the shape of the solid phase is a particle, a suspension of the particles can be used for the above mixing as the solid phase. When the shape of the solid phase is a container such as a microplate, the above mixing can be performed in the container as the solid phase.

When a suspension of the particles is used as the solid phase, the concentration of the first additive and/or the second additive in the mixed solution containing the solid phase obtained in the forming a complex is 1 µM or more and 1000 µM or less, and preferably 10 µM or more and 271 µM or less. When the solid phase is a particle, the mixed solution containing the solid phase obtained in the forming a complex is a liquid containing lipoprotein, labeled sterol, a first additive and/or a second additive, a first capture body, and particles. When using the second capture body, the mixed solution containing the solid phase obtained in the forming a complex is a liquid containing lipoprotein, labeled sterol, a first additive and/or a second additive, a first capture body, a second capture body described later, and particles. When the solid phase is a container, the mixed solution containing the solid phase obtained in the forming a complex is a liquid containing lipoprotein, labeled sterol, a first additive and/or a second additive, and a first capture body in the container. When using the second capture body, the mixed solution containing the solid phase obtained in the forming a complex is a liquid containing lipoprotein, labeled sterol, a first additive and/or a second additive, a first capture body, and a second capture body in the container.

The mixed solution containing the solid phase obtained in the forming a complex is, specifically, a mixed solution of a sample containing lipoprotein, a solution containing labeled sterol, a liquid reagent containing a first additive and/or a second additive, a solution containing a first capture body, and a suspension of particles. When using the second capture body, the mixed solution is a mixed solution of a sample containing lipoprotein, a solution containing labeled sterol, a liquid reagent containing a first additive and/or a second additive, a solution containing a first capture body, a solution containing a second capture body, and a suspension of particles. The liquid reagent may be a reagent containing a first additive and/or a second additive and labeled cholesterol. When the concentration of the first additive and/or the second additive in the mixed solution containing the solid phase obtained in the forming a complex is within the above range, variation of measured values is further reduced.

### (Washing)

**In** this embodiment, washing of removing unreacted free components may be performed between the preparing the lipoprotein incorporating labeled sterol and the measuring ability to uptake sterol described later. This washing includes B/F separation and washing of the complex. The unreacted free components are components that do not constitute a complex containing lipoprotein incorporating labeled sterol. Examples thereof include free labeled sterol that has not been incorporated into lipoprotein, free first capture body that has not bound to lipoprotein, and the like. The means for B/F separation is not particularly limited, but the complex and the unreacted free components can be separated by recovering only the complex by, for example, ultracentrifugation. **In** a case where the complex is formed on a solid phase, when the solid phase is particles, the complex and the unreacted free components can be separated by recovering the particles by centrifugation or magnetic separation, and removing the supernatant. When the solid phase is a container such as a microplate or a microtube, the complex and the unreacted free components can be separated by removing a liquid containing the unreacted free components.

After removing the unreacted free components, the recovered complex can be washed with a suitable aqueous medium. Examples of the aqueous medium include water, physiological saline, PBS, Tris-HCl, Good buffers, and the like. The aqueous medium used for washing preferably contains a surfactant having no cyclic structure. For example, a surfactant having no cyclic structure may be dissolved in the aqueous medium to prepare a washing solution, and the recovered complex may be washed with the washing solution. When a solid phase is used, the solid phase that has captured the complex may be washed with a washing solution. Specifically, a washing solution is added to the recovered complex or the solid phase that has captured the complex, and B/F separation is performed again.

### (Measuring ability to uptake sterol)

In the measurement method of this embodiment, after preparing lipoprotein incorporating labeled sterol, an ability to uptake labeled sterol by lipoproteins is measured. Measurement of ability to uptake sterol is performed by detecting a signal derived from labeled sterol incorporated into lipoprotein. Since this signal reflects the amount of labeled sterol incorporated into lipoprotein, a detection result of the signal is an indicator of lipoprotein's ability to uptake sterol.

The phrase "detecting a signal" herein includes qualitatively detecting the presence or absence of a signal, quantifying a signal intensity, and semi-quantitatively detecting the intensity of a signal in a plurality of stages, such as "not generating signal", "weak", and "strong". In this embodiment, it is preferable to quantify the signal intensity to obtain a measured value.

The signal derived from the labeled sterol incorporated into lipoprotein may be a signal directly generated from the labeled sterol. The signal can be detected, for example, when using labeled sterol having a signal generating substance as the first label. That is, the uptake ability can be measured by detecting the signal generated from the first label in the labeled sterol incorporated into lipoprotein. For example, in the case of using a fluorescently labeled sterol, a fluorescence intensity should be measured. Methods for measuring fluorescence intensity themselves are known in the art. For example, a fluorescence intensity generated from the complex can be measured by using known measuring devices such as a spectrofluorimeter and a fluorescence plate reader. The excitation wavelength and the fluorescence wavelength can be appropriately determined according to the type of fluorescently labeled cholesterol used. For example, when the fluorescently labeled cholesterol of the formula (V) is used, the excitation wavelength should be determined from the range of 470 to 490 nm, and the fluorescence wavelength should be determined from the range of 525 to 550 nm.

### (Forming a complex of labeled sterol and second capture body)

The signal derived from the labeled sterol incorporated into lipoprotein may be a signal generated when detecting the labeled sterol incorporated into lipoprotein. In order to detect the labeled sterol incorporated into lipoprotein, the measurement method of this embodiment preferably includes, between the preparing and the measuring, mixing the lipoprotein incorporating labeled sterol, a first capture body, and a second capture body that binds to labeled sterol and forming a complex containing the lipoprotein incorporating labeled sterol, the first capture body and the second capture body. The labeled sterol is preferably tagged sterol, and more preferably tagged cholesterol. The second capture body is preferably a substance that specifically binds to the tag.

The detection principle of the tagged cholesterol incorporated into lipoprotein is as follows. When cholesterol is incorporated into lipoprotein, the cholesterol normally migrates from a surface layer to a central part of the lipoprotein particle. In the tagged cholesterol, it is the cholesterol moiety that is incorporated into lipoprotein, and the tag is considered to be exposed on an outer surface of the lipoprotein. The "outer surface of the lipoprotein" refers to the outer surface of the lipoprotein particles. The "exposed on an outer surface" means that both existing on the outer surface of the lipoprotein and protruding from the outer surface of the lipoprotein. In this embodiment, the tag exposed on the outer surface is brought into contact with a second capture body that specifically binds to the tag to form a complex. Then, cholesterol incorporated into lipoprotein is detected by detecting the second capture body in this complex.

The substance that specifically binds to the tag can be appropriately determined according to the type of the tag. For example, referring to a combination of the above-mentioned tag and a substance capable of specifically binding to the tag, the substance can be selected from an antibody, a ligand receptor, an oligonucleotide, biotin, avidin, a histidine tag or nickel, GST, glutathione, and the like. Among them, an antibody that specifically binds to the tag is preferred. The antibody may be a commercially available antibody or an antibody prepared by a method known in the art. The antibody may be a monoclonal antibody or a polyclonal antibody. The origin and isotype of the antibody are not particularly limited, and are similar to those described for the anti-HDL antibody. Fragments of antibodies and derivatives thereof may be used, and examples thereof include Fab fragments, F(ab')2 fragments, and the like.

The measurement method of this embodiment more preferably includes, between the forming a complex and the measuring, mixing a complex of the lipoprotein incorporating labeled sterol and a first capture body with a second capture body that binds to labeled sterol and forming a complex containing the lipoprotein incorporating labeled sterol, the first capture body and the second capture body. In particular, it is preferable to form a complex of a lipoprotein incorporating labeled sterol and a first capture body on a solid phase and then mix the complex immobilized on the solid phase with a second capture body. As a result, a complex of the first capture body, the lipoprotein incorporating labeled sterol and the second capture body is formed. In this complex, the lipoprotein incorporating labeled sterol is sandwiched between the first capture body and the second capture body. In this embodiment, the complex of the first capture body, the lipoprotein incorporating labeled sterol and the second capture body is also referred to as a "sandwich complex".

The conditions of temperature and time in the contact between the labeled sterol and the second capture body are not particularly limited. For example, a mixture of a solution containing the complex of the lipoprotein and the first capture body and a solution containing the second capture body may be incubated at 4 to 60°C, preferably 25 to 42°C, for 1 second to 24 hours, preferably 1 minute to 2 hours. During the incubation, the mixture may be allowed to stand, or may be stirred or shaken.

### (Second label)

The second capture body is preferably labeled with a second label. When the second capture body is labeled with a second label, ability to uptake sterol can be measured by detecting a signal derived from the second label in the complex of the labeled sterol and the second capture body. The second label may be a signal generating substance, or a substance that catalyzes a reaction of other substances to generate a detectable signal can be used. Examples of the signal generating substance include fluorescent substances, radioactive isotopes, and the like. Examples of the substance that catalyzes the reaction of other substances to generate a detectable signal include enzymes. Examples of the enzyme include alkaline phosphatase, peroxidase, β-galactosidase, glucose oxidase, tyrosinase, acid phosphatase, luciferase, and the like. Examples of the fluorescent substances include fluorescent dyes such as fluorescein isothiocyanate (FITC), rhodamine and Alexa Fluor (registered trademark) and cyanine dyes, fluorescent proteins such as GFP, and the like. Examples of the radioactive isotopes include ¹²⁵I, ¹⁴C, ³²P, and the like. Among them, an enzyme is preferable, and alkaline phosphatase and peroxidase are particularly preferable.

Labeling of the second capture body with the second label can be performed by directly or indirectly binding the second label to the second capture body. For example, the second label can be directly bonded to the second capture body using a commercially available labeling kit or the like. The second label may be indirectly bound to the second capture body by using a secondary antibody obtained by labeling an antibody capable of specifically binding to the second capture body with the second label. In this embodiment, the second capture body to which the second label is bound may be used. Alternatively, a second capture body and an antibody against the second capture body to which a second label is bound may be used.

In this embodiment, the washing of removing unreacted free components may be performed before signal detection. Examples of the unreacted free components include a free second capture body that did not bind to the tag, a free second label that did not bind to the second capture body, and the like. Specific washing method and washing solution are similar to those in the above-mentioned washing.

### (Detection of signal derived from second label)

Methods for detecting a signal derived from the second label themselves are known in the art. In this embodiment, a suitable measurement method can be selected according to the type of signal derived from the second label. For example, when the second label is an enzyme, signals such as light and color generated by reacting an enzyme and a substrate for the enzyme can be measured by using a known apparatus. Examples of the measuring device include a spectrophotometer, a luminometer, and the like.

The substrate of the enzyme can be appropriately selected from known substrates according to the type of the enzyme. For example, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB). When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate.

When the second label is a radioactive isotope, radiation as a signal can be measured using a known apparatus such as a scintillation counter. When the second label is a fluorescent substance, fluorescence as a signal can be measured using a known apparatus such as a fluorescence microplate reader. The excitation wavelength and the fluorescence wavelength can be appropriately determined according to the type of fluorescent substance used.

In the measurement method of this embodiment, each of the above-mentioned steps is performed in vitro. Each of the above-mentioned steps is performed in a substantially cell-free system. The cell-free system substantially means that cells are not positively added for the purpose of being used to measure the lipoprotein's ability to uptake sterol. For example, in the conventional measurement method of cholesterol efflux function, cholesterol-accumulated cells such as macrophage are used. However, since the labeled sterol is directly incorporated into the lipoprotein in the sample in the measurement method of this embodiment, it is not necessary to use such cells. Even when the sample contains cells derived from a subject, it is considered that the cells themselves hardly affect the lipoprotein's ability to uptake labeled sterol, and the measurement method is considered to be cell-free system.

### [2. Reagent for Measuring Lipoprotein's Uptake Ability, Stabilizing Reagent and Reagent Kit]

In another embodiment, a reagent used in the measurement method of this embodiment is provided. That is, a reagent for measuring lipoprotein's uptake ability (hereinafter, also referred to as "measurement reagent") containing labeled sterol and a first additive and/or a second additive is provided. In addition, a stabilizing reagent for measuring lipoprotein's uptake ability (hereinafter, also referred to as "stabilizing reagent") containing a first additive and/or a second additive is provided. The details of the labeled sterol and the first and second additives are as mentioned above. As used herein, the term "stabilizing reagent" refers to a reagent that improves reproducibility of measurement. The "measurement reagent" of this embodiment is a reagent containing labeled sterol among "stabilizing reagents".

The first additive may be, for example, a compound represented by the formula (I). Examples of the first additive represented by the formula (I) include linear unsaturated aliphatic amides, unsaturated aliphatic alcohols and unsaturated aliphatic amines having one carbon-carbon double bond and having 8 or more and 30 or less carbon atoms. Preferred first additives are oleic acid amide, erucic acid amide, oleyl alcohol, palmitoleyl alcohol, cis-4-decen-1-ol, elaidyl alcohol and oleylamine.

The second additive may be, for example, a saturated aliphatic compound whose main chain has 8 or more and 30 or less carbon atoms. Examples of the saturated aliphatic compound include saturated aliphatic amides, saturated aliphatic alcohols and saturated aliphatic amines having 8 or more and 30 or less carbon atoms in the main chain. The preferred second additive is a linear saturated aliphatic amide, saturated aliphatic alcohol or saturated aliphatic amine having 8 or more and 30 or less carbon atoms. Particularly preferred are stearic acid amide and palmitic acid amide.

The labeled sterol is preferably the above tagged sterol or the above sterol having a signal generating substance. Examples of the tagged sterol include tagged cholesterol represented by the formula (II). Among them, the tagged cholesterol represented by the formulas (V), (VI) and (VII) is preferable. Examples of the sterol having a signal generating substance include fluorescently labeled sterol. Examples of the fluorescently labeled sterol include the fluorescently labeled cholesterol represented by the formula (V).

The measurement reagent and the stabilizing reagent of this embodiment are preferably liquid compositions. Examples of the solvent include aqueous media such as water, physiological saline, PBS, Tris-HCl, and Good buffers. The measurement reagent and the stabilizing reagent of this embodiment are usually stored in a container and provided to a user. The container containing the reagents may be packed in a box or bag and provided to the user. The box or bag may contain a package insert describing how to use the reagents and the like

The measurement reagent of this embodiment may be a one reagent form containing both labeled sterol and a first additive and/or a second additive. Alternatively, the measurement reagent of this embodiment may be a two-reagent form including a first reagent containing labeled sterol and a second reagent containing a first additive and/or a second additive. In a case of the two-reagent form, the first reagent and the second reagent may be provided to the user separately. Alternatively, the first reagent and the second reagent may be packed in a box or the like and provided to the user as a reagent kit. In either case of the one-reagent form or the two-reagent form, it may be provided to the user together with a package insert describing how to use the reagents and the like.

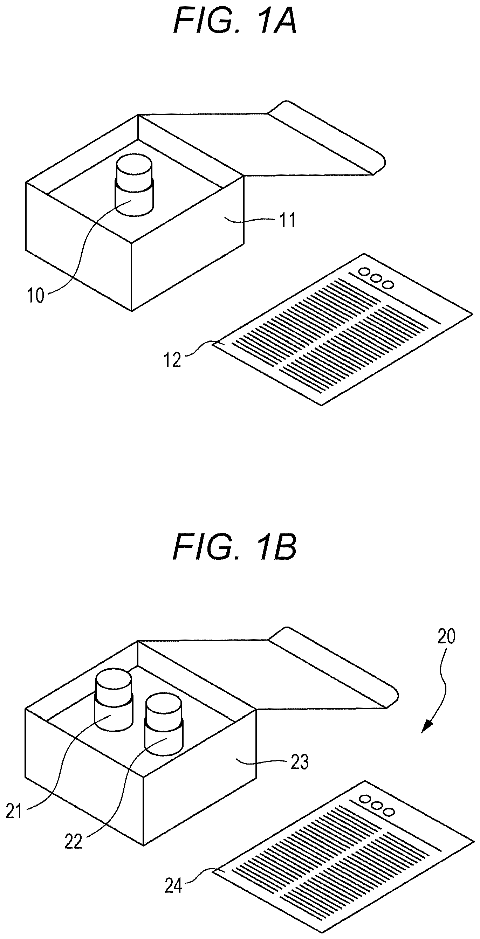

FIG. 1A shows an example of an appearance of a measurement reagent or stabilizing reagent in the one-reagent form. In the figure, 10 denotes a container containing a measurement reagent or a stabilizing reagent, 11 denotes a packing box, and 12 denotes a package insert. FIG. 1B shows an example of an appearance of a reagent kit. This reagent kit includes a first reagent and a second reagent of measurement reagents in the two-reagent form packed in a box. In the figure, 20 denotes a reagent kit, 21 denotes a first container containing a first reagent containing labeled sterol, 22 denotes a second container containing a second reagent containing a first additive and/or a second additive, 23 denotes a packing box, and 24 denotes a package insert.

As to the concentration of the first additive and/or the second additive in each reagent, when the measurement reagent and a sample containing lipoprotein are mixed, or when the stabilizing reagent, a sample containing lipoprotein and a solution containing labeled cholesterol, the concentration of the additive in the mixed solution may be 1 µM or more and 1000 µM or less, and preferably 13 µM or more and 352 µM or less. The concentration of the first additive and/or the second additive in the measurement reagent is, for example, 1 µM or more and 1000 µM or less, and preferably 15 µM or more and 392 µM or less. The concentration of the first additive and/or the second additive in the stabilizing reagent is, for example, 10 µM or more and 10000 µM or less, and preferably 178 µM or more and 1779 µM or less.

As to the concentration of the labeled sterol in the measurement reagent, when the measurement reagent and a sample containing lipoprotein are mixed, the concentration of the labeled sterol in the mixed solution may be 0.1 µM or more and 30 µM or less, and preferably 1 µM or more and 10 µM or less. The concentration of the labeled sterol in the measurement reagent is, for example, 0.2 µM or more and 600 µM or less, and preferably 1 µM or more and 200 µM or less.

The measurement reagent and the stabilizing reagent of this embodiment may further contain a substance useful for measuring lipoprotein's ability to uptake sterol. Examples of the substance useful for measurement include surfactants having no cyclic structure, cyclic oligosaccharides, components that bind to a lipoprotein different from the lipoprotein to be measured, blocking agents, and the like. Details of these substances are as mentioned above.

Examples of the surfactant having no cyclic structure include polyoxyethylene lauryl ether, polyalkylene glycol ethylene oxide adducts, and the like. The concentration of the surfactant in the reagent is not particularly limited, for example, 0.002% (v/v) or more and 10% (v/v) or less, and preferably 0.005% (v/v) or more and 5% (v/v) or less. Examples of the cyclic oligosaccharide include cyclodextrin, methyl-β-cyclodextrin, hydroxypropyl cyclodextrin, and the like. The concentration of the cyclic oligosaccharide in the reagent is not particularly limited, but is, for example, 0.2 mM or more and 20 mM or less, and preferably 1 mM or more and 10 mM or less. Examples of the component that binds to a lipoprotein different from the lipoprotein to be measured include calixarene and the like. The concentration of the component in the reagent is not particularly limited, but when using calixarene, it is, for example, 0.1 µM or more and 0.1 mM or less, and preferably 1 µM or more and 5 µM or less. Examples of the blocking agent include 2-methacryloyloxyethyl phosphoryl choline type polymer. The concentration of the blocking agent in the reagent is not particularly limited, but when using a 2-methacryloyloxyethyl phosphoryl choline type polymer, it is, for example, 0.01% (w/w) or more and 5% (w/w) or less, and preferably 0.05% ( w/w) or more and 2% (w/w) or less.

In another embodiment, use of labeled cholesterol and a first additive and/or a second additive for production of a reagent for measuring lipoprotein's uptake ability is provided. Also, use of a first additive and/or a second additive for production of a stabilizing reagent for measuring lipoprotein's uptake ability is provided. Moreover, use of a first additive and/or a second additive for the above-mentioned method for measuring lipoprotein's uptake ability is provided.

The above-mentioned reagent kit may further include at least one of a first capture body, a second capture body, a solid phase, and a sample dilution reagent. The reagents contained in the reagent kit are preferably stored in separate containers. The details of the labeled sterol, the first and second additives, the first capture body, the second capture body, the solid phase, and the sample dilution reagent are as mentioned above.

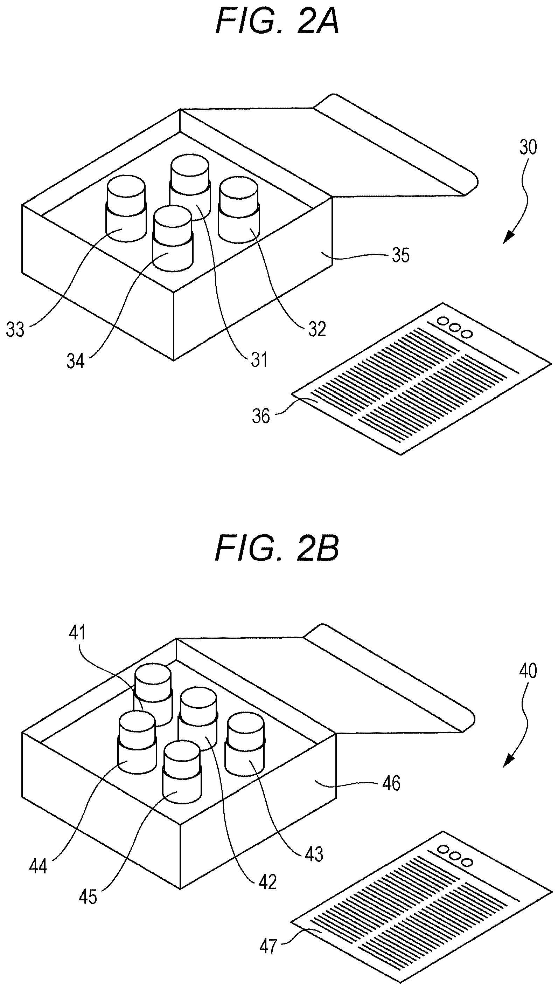

FIG. 2A shows an example of an appearance of a reagent kit of this embodiment. In the figure, 30 denotes a reagent kit, 31 denotes a first container containing a solution containing labeled sterol, 32 denotes a second container containing a liquid reagent containing a first additive and/or a second additive, 33 denotes a third container containing a solution containing a first capture body, 34 denotes a fourth container containing a suspension containing a particle as a solid phase, 35 denotes a packing box, and 36 denotes a package insert.

FIG. 2B shows an example of an appearance of a reagent kit according to another embodiment. In the figure, 40 denotes a reagent kit, 41 denotes a first container containing a solution containing labeled sterol, 42 denotes a second container containing a liquid reagent containing a first additive and/or a second additive, 43 denotes a third container containing a solution containing a first capture body, 44 denotes a fourth container containing a solution containing a second capture body, 45 denotes a fifth container containing a suspension containing a particle as a solid phase, 46 denotes a packing box, and 47 denotes a package insert.

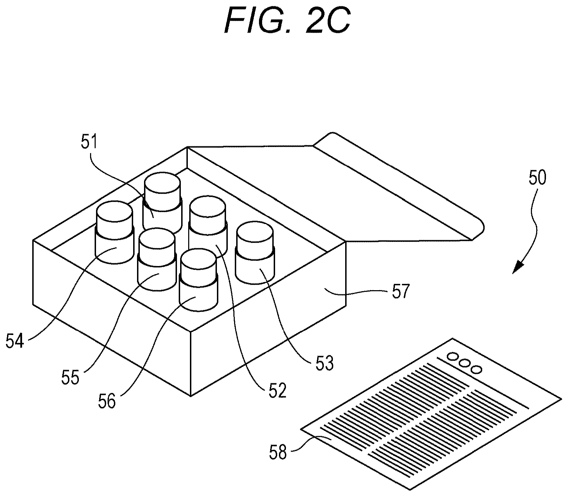

FIG. 2C shows an example of an appearance of a reagent kit according to another embodiment. In the figure, 50 denotes a reagent kit, 51 denotes a first container containing a sample dilution reagent, 52 denotes a second container containing a solution containing labeled sterol, 53 denotes a third container containing a liquid reagent containing a first additive and/or a second additive, 54 denotes a fourth container containing a solution containing a first capture body, 55 denotes a fifth container containing a solution containing a second capture body, 56 denotes a sixth container containing a suspension containing a particle as a solid phase, 57 denotes a packing box, and 58 denotes a package insert.

The reagent kit of this embodiment may include a microplate instead of the container containing a suspension containing a particle. The reagent kit of this embodiment may include a container containing both labeled sterol and a first additive and/or a second additive. The reagent kit of this embodiment may include a container in which a first capture body is immobilized on a particle in advance and a suspension containing the particle to which the first capture body is immobilized is stored. When the second label is an enzyme, the reagent kit of this embodiment may further include a substrate for the enzyme and a buffer necessary for an enzyme reaction.

In another embodiment, use of labeled cholesterol and a first additive and/or a second additive for production of a reagent kit for measuring lipoprotein's uptake ability is provided.

Hereinafter, the present disclosure will be described in more detail by examples, but the present disclosure is not limited to these examples. Hereinafter, "HISCL" refers to a registered trademark of Sysmex Corporation.

### EXAMPLES

### Example 1: Examination of effect of additive (1)

An additive was added to a reaction buffer containing labeled sterol, lipoprotein's ability to uptake cholesterol was measured, and an effect on reproducibility of measurement was examined. Oleic acid amide (Kao Corporation) and erucic acid amide (Tokyo Chemical Industry Co., Ltd.) were used as first additives, and stearic acid amide (Kao Corporation) and palmitic acid amide (Tokyo Chemical Industry Co., Ltd.) were used as second additives. Measurement of uptake ability was performed by a fully automated immunoassay system HI-1000 (Sysmex Corporation) based on ELISA method using a magnetic particle as a solid phase.

### (1) Sample containing lipoprotein

Serum (0.1 mL) of a healthy person was mixed with an equal amount of 22% polyethylene glycol 4000 (Fujifilm Wako Pure Chemical Industries, Ltd.) to obtain a suspension. The resulting suspension was allowed to stand at room temperature for 20 minutes and then centrifuged at 3000 rpm at room temperature for 15 minutes. The resulting supernatant was collected as an HDL fraction. The collected HDL fraction was divided into multiple aliquots and stored frozen until used for measurement.

### (2) Reagent

### (2.1) Sample dilution reagent

Composition of a sample dilution reagent was as follows: 0.02% (v/v) NONION K-230 (NOF Corporation), 0.018% (v/v) Pluronic P84 (BASF), and PIPES buffer solution.

### (2.2) R1 Reagent (solution containing labeled sterol)

Composition of an R1 reagent containing an additive was as follows: 0.003% (w/v) additive, 1% (v/v) Pluronic F68 (Thermo Fisher Scientific, Inc.), 0.3% (v/v) Lipidure (trademark) SF08 (NOF CORPORATION), 1 mM methyl-β-cyclodextrin, 0.05% (v/v) liposome, 0.008% (v/v) NONION K-230, 1 µM biotin-tagged cholesterol, and PBS. The additive was one of oleic acid amide, erucic acid amide, stearic acid amide, and palmitic acid amide. As a control, an R1 reagent having the same composition as above except for containing no additive was prepared. Composition of the liposome was 2 mM dimyristoylphosphatidylglycerol, 2 mM cholesterol, and 4 mM hydrogenated soybean phosphatidylcholine. PBS was prepared by dissolving Phosphate buffered saline tablet (Sigma-Aldrich Co. LLC.) in water. Biotin-tagged cholesterol (PEG7-Biotin) was prepared with reference to Patent Document 1.

### (2.3) R2 Reagent (solid phase on which first capture body is immobilized)

As an R2 reagent, a magnetic particle on which anti-ApoAI antibody was immobilized was prepared. Specifically, the magnetic particle was prepared as follows. WSC (Dojindo) and NHS (Kishida Chemical Co., Ltd.) were added to a magnetic particle, and then an anti-ApoA1 antibody (clone 8E10) was added to bind the anti-ApoA1 antibody to a carboxy group on a surface of the magnetic particle. As a result, a suspension containing a magnetic particle on which anti-ApoAI antibody was immobilized was obtained as the R2 reagent.

### (2.3) R3 Reagent (second capture body having second label)

An alkaline phosphatase (ALP)-labeled streptavidin solution (Vector Laboratories) was used as an R3 reagent.

### (2.4) R4 Reagent (measurement buffer) and R5 reagent (substrate solution)

HISCL R4 reagent (Sysmex Corporation) was used as an R4 reagent. HISCL R5 reagent (Sysmex Corporation) containing CDP-Star (registered trademark) (Applied Biosystems) as a chemiluminescent substrate of ALP was used as an R5 reagent.

### (3) Preparation of lipoprotein incorporating labeled sterol

### (3.1) Dilution of sample

A portion was taken from the HDL fraction, and ApoAI concentration was measured using a kit for ApoAI measurement (N-assay TIA ApoAI-H, Nittobo Medical Co., Ltd.). Specific operations for measuring the concentration were performed according to a manual attached to the kit. After the measurement, the HDL fraction was diluted with a sample dilution reagent to prepare an HDL fraction-containing diluent having an ApoAI concentration of 1,000 ng/mL.

### (3.2) Mixing of HDL and labeled sterols in the presence of additive

The R1 reagent containing each additive was set in a fully automated immunoassay system HI-1000 (Sysmex Corporation). After 1 hour or 70 hours, the R1 reagent (90 µL) containing an additive was added with the HDL fraction-containing diluent (10 µL) and mixed, and the mixture was allowed to stand at 37°C for 1 minute. As a result, HDL was contacted with biotin-added cholesterol in the presence of the additive to promote a biotin-added cholesterol uptake reaction by HDL to obtain a measurement sample. A measurement sample was obtained in the same manner by using an R1 reagent containing no additive.

### (4) Formation of complex of HDL and anti-ApoAl antibody on magnetic particle

The R2 reagent (30 µL) was added to a cuvette containing the measurement sample (100 µL), and the mixture was reacted at 37°C for 6 minutes. The magnetic particle in a reaction solution was magnetically collected, supernatant was removed, and 0.1% (v/v) Pluronic F68/PBS was added to wash the magnetic particle. This washing operation was performed three times in total. Then, the magnetic particle was magnetically collected, and supernatant was removed. The R3 reagent (100 µL) was added to a magnetic particle on which a complex of HDL incorporating biotin-tagged cholesterol and an anti-ApoAl antibody was immobilized, and the mixture was reacted at 37°C for 9 minutes. The magnetic particle in a reaction solution was magnetically collected, supernatant was removed, and 0.1% (v/v) Pluronic F68/PBS was added to wash the magnetic particle. This washing operation was performed three times in total.

### (5) Measurement of HDL's ability to uptake cholesterol

After washing, the magnetic particle was magnetically collected, and supernatant was removed. HISCL R4 reagent (50 µL) and R5 reagent (100 µL) were added to a cuvette containing a magnetic particle, and the mixture was reacted at 37°C for 5 minutes. After the reaction, chemiluminescence intensity was measured. In order to examine the reproducibility of measurement, using the HDL fraction obtained in (1) above, in the same manner as above, the preparation of HDL fraction-containing diluent to the measurement of ability to uptake cholesterol was repeated 10 times. A value of coefficient of variation (CV) was calculated from chemiluminescence intensity obtained by 10 measurements. Table 1 shows CV values (%) of 10 measurements using each additive. In the table, "1 Hour later" and "70 Hours later" indicate elapsed times after the R1 reagent was set in the system.

**[Table 1]**

| Additive | CV (%) | |
|---|---|---|
| | 1 Hour after | 70 Hours after |
| Not added | 16.7 | 14.4 |
| Oleic acid amide | 2.4 | 2.9 |
| Erucic acid amide | 4.7 | 7.1 |
| Stearic acid amide | 11.7 | 11.1 |
| Palmitic acid amide | 11.4 | 12.1 |

### (6) Result

As shown in Table 1, when using the R1 reagent containing no additive, the CV value of 10 measurements was 14% or more. The CV value was reduced as compared with the case of using the R1 reagent containing no additive by using the R1 reagent containing an additive. The smaller the CV value, the smaller the variation of the measured values. In particular, when using an R1 reagent containing oleic acid amide and erucic acid amide, which are compounds containing a hydrocarbon chain having a carbon-carbon unsaturated bond, was used, the CV value was remarkably reduced. From these results, it was shown that oleic acid amide, erucic acid amide, stearic acid amide and palmitic acid amide improve the reproducibility of measuring lipoprotein's ability to uptake sterol. The CV value was not significantly changed between 1 hour after and 70 hours after the R1 reagent was set in the system. Therefore, it was suggested that the reproducibility of measuring uptake ability is hardly affected by the elapsed time after the preparation of the R1 reagent.

### Example 2: Examination of effect of additive (2)

Lipoprotein's ability to uptake cholesterol was measured using a reagent containing an additive, and an effect on reproducibility of measurement was examined. Oleic acid amide (Kao Corporation), palmitoleyl alcohol (Sigma-Aldrich Co. LLC.) and oleylamine (Tokyo Chemical Industry Co., Ltd.) were used as additives. Measurement of uptake ability was performed in the same manner as in Example 1.

### (1) Sample and reagents

The HDL fraction prepared in Example 1 was used as a sample containing lipoprotein. A sample dilution reagent, R2 reagent, R3 reagent, R4 reagent and R5 reagent were the same as those in Example 1. Composition of an R1 reagent containing an additive was as follows: 0.005% (w/v) additive, 1% (v/v) Pluronic F68, 0.3% (v/v) Lipidure (trademark) SF08, 1 mM methyl-β-cyclodextrin, 0.05% (v/v) liposome, 0.008% (v/v) NONION K-230, 1 µM biotin-tagged cholesterol, and PBS. The additive was one of oleic acid amide, palmitoleyl alcohol, and oleylamine. Composition of the liposome was the same as that in Example 1. An R1 reagent containing no additive was prepared in the same manner as in Example 1.

### (2) Preparation of lipoprotein incorporating labeled sterol

The HDL fraction was diluted with a sample dilution reagent to prepare an HDL fraction-containing diluent having an ApoAI concentration of 1,000 ng/mL, in the same manner as in Example 1. The R1 reagent containing each additive was set in HI-1000. After 12 days, the R1 reagent and the HDL fraction-containing diluent were mixed in the same manner as in Example 1 to obtain a measurement sample. A measurement sample was obtained in the same manner by using an R1 reagent containing no additive.

### (3) Formation of complex on solid phase and measurement of uptake ability

The measurement sample was reacted with the R2 reagent, and a magnetic particle was magnetically collected and washed, in the same manner as in Example 1. Then, the magnetic particle on which a complex was immobilized was reacted with the R3 reagent, and the magnetic particle was magnetically collected and washed. After washing, the magnetic particle was magnetically collected, and supernatant was removed. The magnetic particle was reacted with HISCL R4 reagent and R5 reagent, and chemiluminescence intensity was measured, in the same manner as in Example 1. In order to examine the reproducibility of measurement, the preparation of HDL fraction-containing diluent to the measurement of ability to uptake cholesterol was repeated 10 times. The CV value (%) was calculated from chemiluminescence intensity obtained by 10 measurements. Table 2 shows CV values (%) of 10 measurements using each additive. In the table, "12 Days later" indicates elapsed time after the R1 reagent was set in the system.

**[Table 2]**

| Additive | CV (%) |
|---|---|
| | 12 Days after |
| Not added | 14.8 |
| Oleic acid amide | 3.2 |
| Palmitoleyl alcohol | 5.1 |
| Oleylamine | 6.6 |

### (4) Result

From Table 2, as in the case of the results of Example 1, the CV value of 10 measurements was remarkably reduced by using the R1 reagent containing oleic acid amide. Even when using an R1 reagent containing palmitoleyl alcohol and oleylamine was used as an additive, the CV value was remarkably reduced. From these results, it was shown that additive, not only an acid amide containing a hydrocarbon chain having a carbon-carbon unsaturated bond, but also an alcohol and amine containing such a hydrocarbon chain, improve the reproducibility of measuring lipoprotein's ability to uptake sterol.

### Example 3: Examination of effect of additive (3)

Lipoprotein's ability to uptake cholesterol was measured using a reagent containing an additive, and an effect on reproducibility of measurement was examined. Oleic acid amide (Kao Corporation), oleyl alcohol (Tokyo Chemical Industry Co., Ltd.), cis-4-decen-1-ol (Tokyo Chemical Industry Co., Ltd.) and elaidyl alcohol (Tokyo Chemical Industry Co., Ltd.) were used as additives. Measurement of uptake ability was performed in the same manner as in Example 1.

### (1) Sample and reagents

The HDL fraction prepared in Example 1 was used as a sample containing lipoprotein. A sample dilution reagent, R2 reagent, R3 reagent, R4 reagent and R5 reagent were the same as those in Example 1. Composition of an R1 reagent containing an additive was as follows: 0.005% (w/v) additive, 1% (v/v) Pluronic F68, 0.3% (v/v) Lipidure (trademark) SF08, 1 mM methyl-β-cyclodextrin, 0.05% (v/v) liposome, 0.008% (v/v) NONION K-230, 1 µM biotin-tagged cholesterol, and PBS. The additive was one of oleic acid amide, oleyl alcohol, cis-4-decen-1-ol, and elaidyl alcohol. Composition of the liposome was the same as that in Example 1. An R1 reagent containing no additive was prepared in the same manner as in Example 1.

### (2) Preparation of lipoprotein incorporating labeled sterol

The HDL fraction was diluted with a sample dilution reagent to prepare an HDL fraction-containing diluent having an ApoAI concentration of 1,000 ng/mL, in the same manner as in Example 1. The R1 reagent containing each additive was set in HI-1000. After 40 hours, the R1 reagent and the HDL fraction-containing diluent were mixed in the same manner as in Example 1 to obtain a measurement sample. A measurement sample was obtained in the same manner by using an R1 reagent containing no additive.

### (3) Formation of complex on solid phase and measurement of uptake ability

The measurement sample was reacted with the R2 reagent, and a magnetic particle was magnetically collected and washed, in the same manner as in Example 1. Then, the magnetic particle on which a complex was immobilized was reacted with the R3 reagent, and the magnetic particle was magnetically collected and washed. After washing, the magnetic particle was magnetically collected, and supernatant was removed. The magnetic particle was reacted with HISCL R4 reagent and R5 reagent, and chemiluminescence intensity was measured, in the same manner as in Example 1. In order to examine the reproducibility of measurement, the preparation of HDL fraction-containing diluent to the measurement of ability to uptake cholesterol was repeated 10 times. The CV value (%) was calculated from chemiluminescence intensity obtained by 10 measurements. Table 3 shows CV values (%) of 10 measurements using each additive. In the table, "40 Hours later" indicates elapsed time after the R1 reagent was set in the system.

**[Table 3]**

| Additive | CV (%) |
|---|---|
| | 40 Hours after |
| Not added | 21.7 |
| Oleic acid amide | 1.1 |
| Oleyl alcohol | 5.2 |
| Cis-4-decen-1-ol | 7.5 |
| Elaidyl alcohol | 4.9 |

### (4) Result

From Table 3, as in the case of the results of Example 1, the CV value of 10 measurements was remarkably reduced by using the R1 reagent containing oleic acid amide. Even when using an R1 reagent containing oleyl alcohol, cis-4-decen-1-ol and elaidyl alcohol as an additive, the CV value was remarkably reduced. Oleyl alcohol and elaidyl alcohol are geometric isomers.

From the above results, it was suggested that the compound containing a hydrocarbon chain having a carbon-carbon unsaturated bond may be in either a cis form or a trans form. Cis-4-decen-1-ol has a hydrocarbon chain of 10 carbon atoms, and the additives other than cis-4-decen-1-ol have a hydrocarbon chain of 18 carbon atoms. From the above results, it was suggested that the hydrocarbon chain having a carbon-carbon unsaturated bond only needs to have about 10 carbon atoms in the main chain. For comparison, Table 4 summarizes the results (CV value (%)) of Examples 1 to 3.

**[Table 4]**

| Additive | | CV (%) | | | |
|---|---|---|---|---|---|
| | | 1 Hour after | 40 Hours after | 70 Hours after | 12 Days after |
| Not added | | 16.7 | 21.7 | 14.4 | 14.8 |
| Erucic acid amide | C22 Unsaturated aliphatic amide | 4.7 | - | 7.1 | - |
| Oleic acid amide | C18 Unsaturated aliphatic amide | 2.4 | 1.1 | 2.9 | 3.2 |
| Oleyl alcohol | C18 Unsaturated aliphatic alcohol (cis) | - | 5.2 | - | - |
| Elaidyl alcohol | C18 Unsaturated aliphatic alcohol (trans) | - | 4.9 | - | - |
| Palmitoleyl alcohol | C14 Unsaturated aliphatic alcohol | - | - | - | 5.1 |
| Cis-4-decen-1-ol | C10 Unsaturated aliphatic alcohol | - | 7.5 | - | - |
| Oleylamine | C18 Unsaturated aliphatic amine | - | - | - | 6.6 |
| Stearic acid amide | C18 Saturated aliphatic amide | 11.7 | - | 11.1 | - |
| Palmitic acid amide | C16 Saturated aliphatic amide | 11.4 | - | 12.1 | - |

### Example 4: Addition of additive to sample dilution reagent

It was examined whether the reproducibility of measuring lipoprotein's ability to uptake cholesterol was improved even by adding an additive to a sample dilution reagent, as in the case of adding an additive to an R1 reagent. Oleic acid amide (Kao Corporation) was used at various concentrations as an additive. Measurement of uptake ability was performed in the same manner as in Example 1.

### (1) Sample and reagents

The HDL fraction prepared in Example 1 was used as a sample containing lipoprotein. Composition of a sample dilution reagent containing an additive was as follows: 0.005% (w/v), 0.01% (w/v) or 0.02% (w/v) oleic acid amide, 0.02% (v/v) NONION K-230 (NOF CORPORATION), 0.018% (v/v) Pluronic P84 (BASF), and PIPES buffer solution. As the R1 reagent, the R1 reagent containing no additive was prepared in the same manner as in Example 1. A R2 reagent, R3 reagent, R4 reagent and R5 reagent were the same as those in Example 1.

### (2) Preparation of lipoprotein incorporating labeled sterol

The HDL fraction was diluted in the same manner as in Example 1 except that a sample dilution reagent containing an additive was used to prepare an HDL fraction-containing diluent having an ApoAI concentration of 1,000 ng/mL. The R1 reagent was set in HI-1000 (Sysmex Corporation). After 24 hours, the R1 reagent and the HDL fraction-containing diluent were mixed in the same manner as in Example 1 to obtain a measurement sample.

### (3) Formation of complex on solid phase and measurement of uptake ability

The measurement sample was reacted with the R2 reagent, and a magnetic particle was magnetically collected and washed, in the same manner as in Example 1. Then, the magnetic particle on which a complex was immobilized was reacted with the R3 reagent, and the magnetic particle was magnetically collected and washed. After washing, the magnetic particle was magnetically collected, and supernatant was removed. The magnetic particle was reacted with HISCL R4 reagent and R5 reagent, and chemiluminescence intensity was measured, in the same manner as in Example 1. In order to examine the reproducibility of measurement, the preparation of HDL fraction-containing diluent to the measurement of ability to uptake cholesterol was repeated 5 times. The CV value (%) was calculated from chemiluminescence intensity obtained by 5 measurements. Table 5 shows CV values (%) of 5 measurements using each additive.

**[Table 5]**

| Additive | CV (%) |
|---|---|
| 0.005% (w/v) Oleic acid amide | 4.5 |
| 0.01% (w/v) Oleic acid amide | 4.9 |
| 0.02% (w/v) Oleic acid amide | 5.2 |

### (4) Result

From Table 5, the CV value was remarkably reduced by using the sample dilution reagent containing oleic acid amide. Therefore, it was shown that even when the additive was added to the sample dilution reagent, the reproducibility of measuring lipoprotein's ability to uptake sterol was improved.

### Example 5: Examination of concentration of additive

Optimum concentration of additive to improve the reproducibility of measuring uptake ability was examined. Oleic acid amide (Kao Corporation) and erucic acid amide (Tokyo Chemical Industry Co., Ltd.) as additives were each added to an R1 reagent or a sample dilution reagent at various concentrations. Measurement of uptake ability was performed in the same manner as in Example 1.

### (1) Sample and reagents

The HDL fraction prepared in Example 1 was used as a sample containing lipoprotein. A R2 reagent, R3 reagent, R4 reagent and R5 reagent were the same as those in Example 1.

Composition of a sample dilution reagent containing oleic acid amide was as follows: 177.9 µM, 355.9 µM, 711.7 µM, 1067.6 µM or 1779.4 µM oleic acid amide, 0.02% (v/v) NONION K-230, 0.018% (v/v) Pluronic P84, and a PIPES buffer solution. A sample dilution reagent containing no additive was prepared in the same manner as in Example 1.

Composition of an R1 reagent containing oleic acid amide was as follows: 26.7 µM, 53.4 µM, 106.8 µM, 160.1 µM, 249.1 µM, 320.3 µM or 391.5 µM oleic acid amide, 1% (v/v) Pluronic F68, 0.3% (v/v) Lipidure (trademark) SF08, 1 mM methyl-β-cyclodextrin, 0.05% (v/v) liposome, 0.008% (v/v) NONION K-230, 1 µM biotin-tagged cholesterol, and PBS. Composition of the liposome was the same as that in Example 1.

Composition of an R1 reagent containing erucic acid amide was as follows: 14.8 µM, 44.4 µM, 147.9 µM, 221.9 µM or 295.9 µM erucic acid amide, 1% (v/v) Pluronic F68, 0.3% (v/v) Lipidure (trademark) SF08, 1 mM methyl-β-cyclodextrin, 0.05% (v/v) liposomes, 0.008% (v/v) NONION K-230, 1 µM biotin-tagged cholesterol, and PBS. Composition of the liposome was the same as that in Example 1.

### (2) Preparation of lipoprotein incorporating labeled sterol

### (2.1) In case of using R1 reagent containing additive

The HDL fraction was diluted with a sample dilution reagent containing no additive to prepare an HDL fraction-containing diluent having an ApoAI concentration of 1,000 ng/mL, in the same manner as in Example 1. The R1 reagent containing each additive was set in HI-1000. After 26 hours, the HDL was contacted with biotin-added cholesterol in the presence of an additive in the same manner as in Example 1 to obtain a measurement sample.

### (2.2) In case of using sample dilution reagent containing additive

The HDL fraction was diluted in the same manner as in Example 1 except that a sample dilution reagent containing oleic acid amide was used to prepare an HDL fraction-containing diluent having an ApoAI concentration of 1,000 ng/mL. The R1 reagent containing no additive was set in HI-1000 (Sysmex Corporation). After 24 hours, the R1 reagent and the HDL fraction-containing diluent were mixed in the same manner as in Example 1 to obtain a measurement sample.

### (3) Formation of complex on solid phase and measurement of uptake ability

The measurement sample was reacted with the R2 reagent, and a magnetic particle was magnetically collected and washed, in the same manner as in Example 1. Then, the magnetic particle on which a complex was immobilized was reacted with the R3 reagent, and the magnetic particle was magnetically collected and washed. After washing, the magnetic particle was magnetically collected, and supernatant was removed. The magnetic particle was reacted with HISCL R4 reagent and R5 reagent, and chemiluminescence intensity was measured, in the same manner as in Example 1. In order to examine the reproducibility of measurement, the preparation of HDL fraction-containing diluent to the measurement of ability to uptake cholesterol was repeated 5 times. The CV value (%) was calculated from chemiluminescence intensity obtained by 5 measurements. Tables 6 to 8 show CV values (%) of measurements using each reagent. In the tables, "concentration after addition of R1 reagent" indicates concentration of the additive in the measurement sample which is a mixed solution of the sample containing lipoprotein (HDL fraction), the sample dilution reagent and the R1 reagent. "Concentration after addition of R2 reagent" indicates concentration of the additive in the mixed solution of the measurement sample and the R2 reagent.

**[Table 6]**

| R1 Reagent containing oleic acid amide | | | | | | | |
|---|---|---|---|---|---|---|---|
| Concentration in R1 reagent (µM) | 26.7 | 53.4 | 106.8 | 160.1 | 249.1 | 320.3 | 391.5 |
| Concentration after addition of R1 reagent (µM) | 24.0 | 48.0 | 96.1 | 144.1 | 224.2 | 288.3 | 352.3 |
| Concentration after addition of R2 reagent (µM) | 18.5 | 37.0 | 73.9 | 110.9 | 172.5 | 221.7 | 271.0 |
| CV (%) | 8.6 | 6.3 | 3.7 | 3.9 | 2.1 | 1.8 | 1.9 |

**[Table 7]**

| R1 Reagent containing erucic acid amide | | | | | |
|---|---|---|---|---|---|
| Concentration in R1 reagent (µM) | 14.8 | 44.4 | 147.9 | 221.9 | 295.9 |
| Concentration after addition of R1 reagent (µM) | 13.3 | 39.9 | 133.1 | 199.7 | 266.3 |
| Concentration after addition of R2 reagent | 10.2 | 30.7 | 102.4 | 153.6 | 204.8 |
| CV (%) | 5.6 | 6.5 | 6.2 | 5.1 | 7.7 |

**[Table 8]**

| Sample dilution reagent containing oleic acid amide | | | | | |
|---|---|---|---|---|---|
| Concentration in sample dilution reagent (µM) | 177.9 | 355.9 | 711.7 | 1067.6 | 1779.4 |
| Concentration after addition of R1 reagent (µM) | 17.8 | 35.6 | 71.2 | 106.8 | 177.9 |
| Concentration after addition of R2 reagent (µM) | 13.7 | 27.4 | 54.7 | 82.1 | 136.9 |
| CV (%) | 4.5 | 4.9 | 5.2 | 7.8 | 9.4 |

From the results shown in Tables 6 to 8, the effect of improving the reproducibility of measurement was recognized at any concentration.

## Claims

1. A method for measuring lipoprotein's uptake ability, comprising:
preparing lipoprotein incorporating labeled sterol by mixing lipoprotein in a sample with the labeled sterol, in a presence of at least one compound selected from the group consisting of: a compound comprising a hydrocarbon chain having at least one carbon-carbon unsaturated bond (excluding a sterol); and a saturated aliphatic compound having no phosphodiester bond; and
measuring an ability to uptake the labeled sterol, based on a label of the labeled sterol incorporated into the lipoprotein,
wherein the compound comprising the hydrocarbon chain is at least one selected from the group consisting of oleic acid amide, erucic acid amide, oleyl alcohol, palmitoleyl alcohol, cis-4-decen-1-ol, elaidyl alcohol, and oleylamine,
wherein the saturated aliphatic compound is at least one selected from the group consisting of stearic acid amide and palmitic acid amide.

2. The method according to claim 1, wherein the mixing in the preparing is performed by mixing the sample comprising the lipoprotein, the labeled sterol, and a liquid reagent comprising said at least one compound selected from the group consisting of: the compound comprising the hydrocarbon chain having at least one carbon-carbon unsaturated bond (excluding the sterol); and the saturated aliphatic compound having no phosphodiester bond.

3. The method according to claim 1, wherein a concentration of the compound comprising the hydrocarbon chain and/or the saturated aliphatic compound in a mixed solution obtained in the preparing is 1 µM or more and 1000 µM or less.

4. The method according to claim 1, wherein the labeled sterol is labeled cholesterol.

5. The method according to claim 4, wherein
the labeled cholesterol is tagged cholesterol represented by a following formula (III): 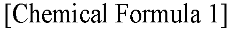 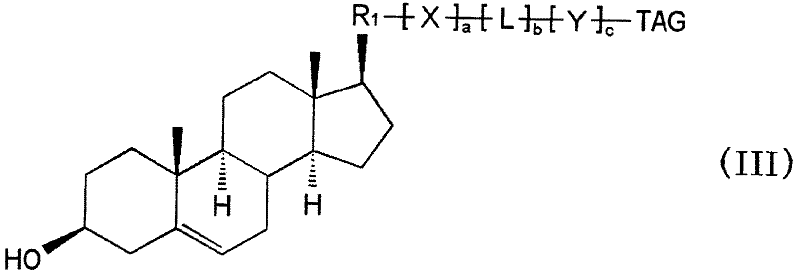
wherein R₁ is an alkylene group having 1 to 6 carbon atoms which optionally have a methyl group,
X and Y are identical or different, and are represented by -R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-,-R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S-, or -S-R₂-, and each R₂ is independently an atomic bonding, an alkylene group having 1 or more and 10 or less carbon atoms which optionally have a substituent, or an arylene group or heteroarylene group having 6 or more and 12 or less carbon atoms which optionally have a substituent, or a cycloalkylene group or heterocycloalkylene group having 3 or more and 8 or less carbon atoms which optionally have a substituent,
L is represented by -(CH₂)_{d}-[R₃-(CH₂)ₑ]_{f}- or -[(CH₂)ₑ-R₃]_{f}-(CH₂)_{d}-, and R₃ is an oxygen atom, a sulfur atom, -NH-, -NH-(C=O)- or -(C=O)-NH-,
TAG is a tag,
a and c are identical or different and are an integer of 0 to 6,
b is 0 or 1,
d and e are identical or different and are an integer of 0 to 12, and
f is an integer of 0 to 24.

6. The method according to claim 1, comprising, between the preparing and the measuring,
mixing the lipoprotein incorporating labeled sterol with a first capture body that binds to the lipoprotein, and
forming a complex comprising the lipoprotein incorporating labeled sterol and the first capture body.

7. The method according to claim 1, comprising, between the preparing and the measuring,
mixing the lipoprotein incorporating labeled sterol, a first capture body that binds to the lipoprotein and a second capture body that binds to the labeled sterol, and
forming a complex comprising the lipoprotein incorporating labeled sterol, the first capture body and the second capture body.

8. The method according to claim 6, wherein the measuring is performed by detecting a signal derived from the labeled sterol incorporated into the lipoprotein.

9. The method according to claim 7, wherein the measuring is performed by detecting the second capture body.

10. The method according to claim 9, wherein the second capture body is labeled with a second label, and the measuring comprises detecting a signal derived from the second label.

11. The method according to claim 10, wherein the second label is an enzyme and the signal is a chemiluminescent signal generated from a reaction product of the enzyme and a substrate.

12. A method for measuring a labeled sterol, comprising:
forming a complex comprising a lipoprotein and the labeled sterol, in a presence of at least one compound selected from the group consisting of: a compound comprising a hydrocarbon chain having at least one carbon-carbon unsaturated bond (excluding a sterol); and a saturated aliphatic compound having no phosphodiester bond; and
measuring the labeled sterol in the complex,
wherein the compound comprising the hydrocarbon chain is at least one selected from the group consisting of oleic acid amide, erucic acid amide, oleyl alcohol, palmitoleyl alcohol, cis-4-decen-1-ol, elaidyl alcohol, and oleylamine,
wherein the saturated aliphatic compound is at least one selected from the group consisting of stearic acid amide and palmitic acid.

13. A method for measuring a labeled sterol, comprising:
forming a complex comprising a lipoprotein and a labeled sterol in a presence of at least one compound selected from the group consisting of: a compound comprising a hydrocarbon chain having at least one carbon-carbon unsaturated bond (excluding a sterol); and a saturated aliphatic compound having no phosphodiester bond;
binding a capture body with a label of the labeled sterol in the complex, the capture body comprising a fluorescent substance or an enzyme; and
measuring a signal resulted from the fluorescent substance of the complex or from an enzymatic reaction by the enzyme of the complex,
wherein the compound comprising the hydrocarbon chain is at least one selected from the group consisting of oleic acid amide, erucic acid amide, oleyl alcohol, palmitoleyl alcohol, cis-4-decen-1-ol, elaidyl alcohol, and oleylamine,
wherein the saturated aliphatic compound is at least one selected from the group consisting of stearic acid amide and palmitic acid amide.

## Patentansprüche

1. Verfahren zum Messen der Aufnahmefähigkeit von Lipoprotein, umfassend:
Herstellen von Lipoprotein, enthaltend markierte Sterol, durch Mischen von Lipoprotein in einer Probe mit dem markierten Sterol in Gegenwart von mindestens einer Verbindung, ausgewählt aus der Gruppe bestehend aus: einer Verbindung, die eine Kohlenwasserstoffkette mit mindestens einer ungesättigten Kohlenstoff-Kohlenstoff-Bindung (ausgenommen ein Sterol) umfasst; und einer gesättigten aliphatischen Verbindung ohne Phosphodiesterbindung; und
Messen der Fähigkeit zur Aufnahme des markierten Sterols, basierend auf einer Markierung des in dem Lipoprotein enthaltenden markierten Sterols,
wobei die Verbindung, die die Kohlenwasserstoffkette umfasst, mindestens eine aus der Gruppe bestehend aus Ölsäureamid, Erucasäureamid, Oleylalkohol, Palmitoleylalkohol, cis-4-Decen-1-ol, Elaidylalkohol und Oleylamin ausgewählt ist, wobei die gesättigte aliphatische Verbindung mindestens eine aus der Gruppe bestehend aus Stearinsäureamid und Palmitinsäureamid ausgewählt ist.

2. Verfahren nach Anspruch 1, wobei das Mischen in der Herstellung durch Mischen der Probe, umfassend das Lipoprotein, des markierten Sterols und eines flüssigen Reagenzes, umfassend mindestens eine Verbindung ausgewählt aus Gruppe bestehend aus: der Verbindung, die die Kohlenwasserstoffkette mit mindestens einer ungesättigten Kohlenstoff-Kohlenstoff-Bindung (ausgenommen das Sterol) umfasst; und der gesättigten aliphatischen Verbindung ohne Phosphodiesterbindung; durchgeführt wird.

3. Verfahren nach Anspruch 1, wobei die Konzentration der Verbindung, umfassend die Kohlenwasserstoffkette und/oder die gesättigte aliphatische Verbindung in einer gemischten Lösung, die in der Herstellung erhalten wird, 1 µM oder mehr und 1000 µM oder weniger beträgt.

4. Verfahren nach Anspruch 1, wobei das markierte Sterol markiertes Cholesterin ist.

5. Verfahren nach Anspruch 4, wobei das markierte Cholesterin markiertes (tagged) Cholesterin ist, das durch die folgende Formel (III) dargestellt wird: 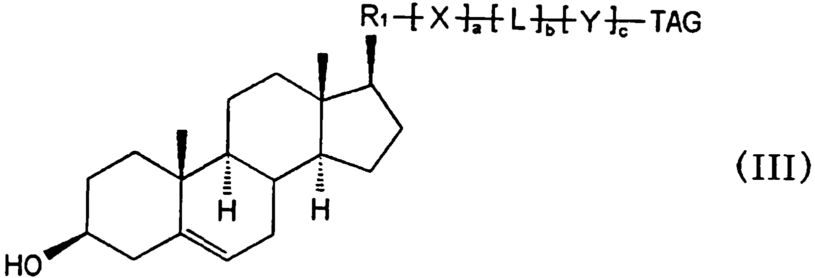
wobei R₁ eine Alkylengruppe mit 1 bis 6 Kohlenstoffatomen ist, die gegebenenfalls eine Methylgruppe aufweisen,
X und Y identisch oder unterschiedlich sind und durch - R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S- oder -S-R₂-, und jedes R₂ unabhängig voneinander eine atomare Bindung, eine Alkylengruppe mit 1 oder mehr und 10 oder weniger Kohlenstoffatomen, die gegebenenfalls einen Substituenten aufweisen, oder eine Arylengruppe oder Heteroarylengruppe mit 6 oder mehr und 12 oder weniger Kohlenstoffatomen, die gegebenenfalls einen Substituenten aufweisen, oder eine Cycloalkylengruppe oder Heterocycloalkylengruppe mit 3 oder mehr und 8 oder weniger Kohlenstoffatomen, die gegebenenfalls einen Substituenten aufweisen, ist,
L durch - (CH₂)_{d}-[R₃-(CH₂)ₑ]_{f}- oder -[(CH₂)ₑ-R₃]_{f}-(CH₂)_{d}- dargestellt wird und R₃ ein Sauerstoffatom, ein Schwefelatom, -NH-, -NH-(C=O)- oder -(C=O)-NH- ist,
TAG ein Tag ist,
a und c identisch oder unterschiedlich sind und eine ganze Zahl von 0 bis 6 darstellen,
b 0 oder 1 ist,
d und e identisch oder unterschiedlich sind und eine ganze Zahl von 0 bis 12 darstellen und f eine ganze Zahl von 0 bis 24 ist.

6. Verfahren nach Anspruch 1, umfassend zwischen der Vorbereitung und der Messung das Mischen des Lipoproteins, enthaltend das markierte Sterol, mit einem ersten Einfangkörper (capture body), der an das Lipoprotein bindet, und das Bilden eines Komplexes, umfassend das markierte Sterol enthaltende Lipoprotein, und den ersten Einfangkörper.

7. Verfahren nach Anspruch 1, umfassend zwischen der Vorbereitung und der Messung das Mischen des Lipoproteins, enthaltend markierte Sterol, eines ersten Einfangkörpers, der an das Lipoprotein bindet, und eines zweiten Einfangkörpers, der an das markierte Sterol bindet, und das Bilden eines Komplexes, umfassend das das markierte Sterol enthaltende Lipoprotein, den ersten Einfangkörper und den zweiten Einfangkörper.

8. Verfahren nach Anspruch 6, wobei die Messung anhand von Detektieren eines Signals durchgeführt wird, das von dem in dem Lipoprotein enthaltenden markierten Sterol abgeleitet ist.

9. Verfahren nach Anspruch 7, wobei die Messung anhand von Detektieren des zweiten Einfangkörpers durchgeführt wird.

10. Verfahren nach Anspruch 9, wobei der zweite Einfangkörper mit einer zweiten Markierung markiert ist und die Messung das Detektieren eines von der zweiten Markierung abgeleiteten Signals umfasst.

11. Verfahren nach Anspruch 10, wobei die zweite Markierung ein Enzym ist und das Signal ein Chemilumineszenzsignal ist, das aus einem Reaktionsprodukt des Enzyms und eines Substrats erzeugt wird.

12. Verfahren zum Messen eines markierten Sterols, umfassend:
Bilden eines Komplexes, umfassend ein Lipoprotein und das markierte Sterol, in Gegenwart von mindestens einer Verbindung, ausgewählt aus der Gruppe bestehend aus: einer Verbindung, die eine Kohlenwasserstoffkette mit mindestens einer ungesättigten Kohlenstoff-Kohlenstoff-Bindung (ausgenommen ein Sterol) umfasst; und einer gesättigten aliphatischen Verbindung ohne Phosphodiesterbindung; und
Messen des markierten Sterols in dem Komplex, wobei die Verbindung, die die Kohlenwasserstoffkette umfasst, mindestens eine aus der Gruppe bestehend aus Ölsäureamid, Erucasäureamid, Oleylalkohol, Palmitoleylalkohol, cis-4-Decen-1-ol, Elaidylalkohol und Oleylamin ausgewählt ist, wobei die gesättigte aliphatische Verbindung mindestens eine aus der Gruppe bestehend aus Stearinsäureamid und Palmitinsäure ausgewählt ist.

13. Verfahren zum Messen eines markierten Sterols, umfassend:
Bilden eines Komplexes, umfassend ein Lipoprotein und ein markiertes Sterol in Gegenwart von mindestens einer Verbindung, ausgewählt aus der Gruppe bestehend aus: einer Verbindung, die eine Kohlenwasserstoffkette mit mindestens einer ungesättigten Kohlenstoff-Kohlenstoff-Bindung (ausgenommen ein Sterol) umfasst; und einer gesättigten aliphatischen Verbindung ohne Phosphodiesterbindung;
Binden eines Einfangkörpers mit einer Markierung des markierten Sterols in dem Komplex, wobei der Einfangkörper eine fluoreszierende Substanz oder ein Enzym umfasst;
und Messen eines Signals, das aus der fluoreszierenden Substanz des Komplexes oder aus einer enzymatischen Reaktion durch das Enzym des Komplexes resultiert,
wobei die Verbindung, die die Kohlenwasserstoffkette umfasst, mindestens eine aus der Gruppe bestehend aus Ölsäureamid, Erucasäureamid, Oleylalkohol, Palmitoleylalkohol, cis-4-Decen-1-ol, Elaidylalkohol und Oleylamin ausgewählt ist, wobei die gesättigte aliphatische Verbindung mindestens eine aus der Gruppe bestehend aus Stearinsäureamid und Palmitinsäureamid ausgewählt ist.

## Revendications

1. Procédé pour la mesure de la capacité d'absorption de lipoprotéine, comprenant :
la préparation d'une lipoprotéine incorporant un stérol marqué en mélangeant une lipoprotéine dans un échantillon avec le stérol marqué, en présence d'au moins un composé choisi dans le groupe consistant en : un composé comprenant une chaîne hydrocarbonée présentant au moins une liaison insaturée carbone-carbone (excluant un stérol) ; et un composé aliphatique saturé ne présentant aucune liaison phosphodiester ; et
la mesure d'une capacité à absorber le stérol marqué, sur la base d'un marqueur du stérol marqué incorporé à la lipoprotéine,
dans lequel le composé comprenant la chaîne hydrocarbonée est au moins un composé choisi dans le groupe consistant en amide d'acide oléique, amide d'acide érucique, alcool oléique, alcool palmitoléique, cis-4-décén-1-ol, alcool élaïdique, et oléylamine,
dans lequel le composé aliphatique saturé est au moins un composé choisi dans le groupe consistant en amide d'acide stéarique et amide d'acide palmitique.

2. Procédé selon la revendication 1, dans lequel le mélange dans la préparation est réalisé en mélangeant l'échantillon comprenant la lipoprotéine, le stérol marqué, et un réactif liquide comprenant ledit au moins un composé choisi dans le groupe consistant en : le composé comprenant la chaîne hydrocarbonée présentant au moins une liaison insaturée carbone-carbone (excluant le stérol) ; et le composé aliphatique saturé ne présentant aucune liaison phosphodiester.

3. Procédé selon la revendication 1, dans lequel une concentration du composé comprenant la chaîne hydrocarbonée et/ou le composé aliphatique saturé dans une solution mixte obtenue dans la préparation est comprise entre 1 µM ou plus et 1000 µM ou moins.

4. Procédé selon la revendication 1, dans lequel le stérol marqué est du cholestérol marqué.

5. Procédé selon la revendication 4, dans lequel le cholestérol marqué est du cholestérol marqué représenté par une formule suivante (III) : 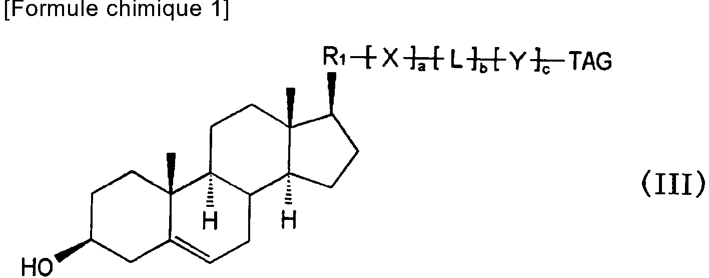
dans lequel R₁ est un groupe alkylène comprenant de 1 à 6 atomes de carbone comprenant facultativement un groupe méthyle,
X et Y sont identiques ou différents, et sont représentés par -R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S-, ou -S-R₂-, et chaque R₂ est indépendamment une liaison atomique, un groupe alkylène comprenant 1 ou plus et 10 ou moins atomes de carbone comprenant facultativement un substituant, ou un groupe arylène ou un groupe hétéroarylène comprenant 6 ou plus et 12 ou moins atomes de carbone comprenant facultativement un substituant, ou un groupe cycloalkylène ou un groupe hétérocycloalkylène comprenant 3 ou plus et 8 ou moins atomes de carbone comprenant facultativement un substituant,
L est représenté par -(CH₂)_{d}-[R₃-(CH₂)ₑ]_{f}- ou -[(CH₂)ₑ-R₃]_{f}-(CH₂)_{d}-, et R₃ est un atome d'oxygène, un atome de soufre, -NH-, -NH-(C=O)- ou -(C=O)-NH-,
TAG est un marqueur,
a et c sont identiques ou différents et sont un nombre entier de 0 à 6,
b est 0 ou 1,
d et e sont identiques ou différents et sont un nombre entier de 0 à 12, et
f est un nombre entier de 0 à 24.

6. Procédé selon la revendication 1, comprenant, entre la préparation et la mesure,
le mélange de la lipoprotéine incorporant un stérol marqué avec un premier corps de capture qui se lie à la lipoprotéine, et
la formation d'un complexe comprenant la lipoprotéine incorporant un stérol marqué et le premier corps de capture.

7. Procédé selon la revendication 1, comprenant, entre la préparation et la mesure,
le mélange de la lipoprotéine incorporant un stérol marqué, un premier corps de capture qui se lie à la lipoprotéine et un second corps de capture qui se lie au stérol marqué, et
la formation d'un complexe comprenant la lipoprotéine incorporant un stérol marqué, le premier corps de capture et le second corps de capture.

8. Procédé selon la revendication 6, dans lequel la mesure est réalisée par détection d'un signal dérivé du stérol marqué incorporé à la lipoprotéine.

9. Procédé selon la revendication 7, dans lequel la mesure est réalisée par détection du second corps de capture.

10. Procédé selon la revendication 9, dans lequel le second corps de capture est marqué avec un second marqueur, et la mesure comprend la détection d'un signal dérivé du second marqueur.

11. Procédé selon la revendication 10, dans lequel le second marqueur est une enzyme et le signal est un signal chimioluminescent généré à partir d'un produit de réaction de l'enzyme et d'un substrat.

12. Procédé pour la mesure d'un stérol marqué, comprenant :
la formation d'un complexe comprenant une lipoprotéine et le stérol marqué, en présence d'au moins un composé choisi dans le groupe consistant en : un composé comprenant une chaîne hydrocarbonée présentant au moins une liaison insaturée carbone-carbone (excluant un stérol) ; et un composé aliphatique saturé ne présentant aucune liaison phosphodiester ; et
la mesure du stérol marqué dans le complexe,
dans lequel le composé comprenant la chaîne hydrocarbonée est au moins un composé choisi dans le groupe consistant en amide d'acide oléique, amide d'acide érucique, alcool oléique, alcool palmitoléique, cis-4-décén-1-ol, alcool élaïdique, et oléylamine,
dans lequel le composé aliphatique saturé est au moins un composé choisi dans le groupe consistant en amide d'acide stéarique et amide d'acide palmitique.

13. Procédé pour la mesure d'un stérol marqué, comprenant :
la formation d'un complexe comprenant une lipoprotéine et un stérol marqué en présence d'au moins un composé choisi dans le groupe consistant en : un composé comprenant une chaîne hydrocarbonée présentant au moins une liaison insaturée carbone-carbone (excluant un stérol) ; et un composé aliphatique saturé ne présentant aucune liaison phosphodiester ;
la liaison d'un corps de capture avec un marqueur du stérol marqué dans le complexe, le corps de capture comprenant une substance fluorescente ou une enzyme ; et
la mesure d'un signal résultant de la substance fluorescente du complexe ou d'une réaction enzymatique par l'enzyme du complexe,
dans lequel le composé comprenant la chaîne hydrocarbonée est au moins un composé choisi dans le groupe consistant en amide d'acide oléique, amide d'acide érucique, alcool oléique, alcool palmitoléique, cis-4-décén-1-ol, alcool élaïdique, et oléylamine,
dans lequel le composé aliphatique saturé est au moins un composé choisi dans le groupe consistant en amide d'acide stéarique et amide d'acide palmitique.
